# EUROPEAN PATENT APPLICATION

(11) **EP 4 697 355 A1**
(43) Date of publication of application: **18.02.2026**
(21) Application number: 24195048.4
(22) Date of filing: 16.08.2024
(51) Int. Cl.: G16H 40/67, H04L 67/12, H04W 12/06

(54) **A DATA TRANSMISSION METHOD, DEVICE, ELECTRONIC EQUIPMENT, AND STORAGE MEDIUM**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application discloses a data transmission method, device, electronic equipment, and storage medium, wherein the data transmission method comprises: receiving health monitoring equipment activation success information and marking the status of the health monitoring equipment as "activated", where the health monitoring equipment activation success information is transmitted by the health monitoring equipment after the designated mobile terminal activates the health monitoring equipment, or transmitted after the base station activates the health monitoring equipment; receiving the associated information of the connectable candidate health monitoring equipment to be connected transmitted by the base station within its coverage; if the connection conditions are determined to be met based on the associated information of the candidate health monitoring equipment to be connected and the status of the candidate health monitoring equipment to be connected, a message that instructs the connection of the candidate health monitoring equipment to be connected will be returned to the base station, so that the base station will be connected to the candidate health monitoring equipment to be connected; receiving the health monitoring data that is transmitted from the base station and collected from the health monitoring equipment.

## Description

### Technical Field

The present application involves artificial intelligence, particularly a data transmission method, device, electronic equipment, and storage medium.

### Background

Health monitoring equipment has been increasingly widely used in the public places such as hospital and nursing home. The health monitoring equipment worn by the monitored object will in real time or regularly collect the health monitoring data of the monitored object such as physiology, biochemistry and vital signs, so as to monitor the state of health of the monitored object. In this technology, the data collected by the health monitoring equipment is transmitted in the following process: the health monitoring application program (APP) is installed in the mobile terminal of the monitored object; the health monitoring equipment is connected to the health monitoring application program in the mobile terminal via Bluetooth communication; the health monitoring application program in the mobile terminal activates the health monitoring equipment and collects the health monitoring data of the monitored object obtained by the health monitoring equipment; the health monitoring application program in the mobile terminal uploads the health monitoring data of the monitored object to the server; the server uses the received health monitoring data to provide personalized health management and diagnosis services to the monitored object.

In the scenarios such as hospital or nursing home, however, certain users may not have a mobile phone or any other mobile terminal equipment, alternatively, certain old aged users do not know how to use the health monitoring application program, so the health monitoring equipment cannot be activated and the health monitoring data cannot be uploaded. In a hospital or nursing home, the movement of the monitored object wearing the health monitoring equipment may cause communication disconnection between the mobile terminal of the monitored object and the health monitoring equipment and consequently the health monitoring data of the monitored object cannot be continuously uploaded in real time.

### Summary of the Invention

In order to solve the problems that activation of health monitoring equipment and uploading of health monitoring data may fail due to the limitations of the prior art and that the communication disconnection between the mobile terminal of the monitored object and the health monitoring equipment may cause the health monitoring data of the monitored object to fail to be uploaded continuously in real time, the embodiment of the present application offers a data transmission method, device, electronic equipment, and storage medium.

In a first aspect, the embodiment of the present application offers a data transmission method implemented at the server, comprising:
receiving health monitoring equipment activation success information and marking the status of the health monitoring equipment as "activated", wherein the health monitoring equipment activation success information is transmitted by the health monitoring equipment after the designated mobile terminal activates the health monitoring equipment, or transmitted after the base station activates the health monitoring equipment;
receiving the associated information of the candidate health monitoring equipment to be connected, wherein the candidate health monitoring equipment to be connected is connectable health monitoring equipment to be connected detected by the base station within its coverage;
returning to the base station a message that instructs the connection of the candidate health monitoring equipment to be connected if the connection conditions are determined to be met based on the associated information of the candidate health monitoring equipment to be connected and the status of the candidate health monitoring equipment to be connected, so that the base station will be connected to the candidate health monitoring equipment to be connected; and
receiving the health monitoring data that is transmitted from the base station and collected from the health monitoring equipment connected to the base station.

In one embodiment, the associated information of the candidate health monitoring equipment to be connected includes the identification information of the candidate health monitoring equipment to be connected and the signal strength between the candidate health monitoring equipment to be connected and the base station;
determining whether the connection conditions are met based on the associated information of the candidate health monitoring equipment to be connected and the status of the candidate health monitoring equipment to be connected, specifically comprising:
searching for the status of the candidate health monitoring equipment to be connected based on the identification of the candidate health monitoring equipment to be connected; and
confirming the connection conditions are met if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the first base station at the moment the equipment was disconnected from the base station to which it was connected last time.

In one embodiment, the associated information of the candidate health monitoring equipment to be connected includes the identification information of the candidate health monitoring equipment to be connected and the signal strength between the candidate health monitoring equipment to be connected and the base station;
determining whether the connection conditions are met based on the associated information of the candidate health monitoring equipment to be connected and the status of the candidate health monitoring equipment to be connected, specifically comprising:
searching for the status of the candidate health monitoring equipment to be connected based on the identification of the candidate health monitoring equipment to be connected; and
confirming the connection conditions are met if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the second base station adjacent to the base station.

In one embodiment, the message that instructs the connection of the candidate health monitoring equipment contains the authentication information of the candidate health monitoring equipment to be connected, so that the base station and the candidate health monitoring equipment to be connected will execute bidirectional authentication based on the authentication information, and they will connect after successful authentication.

In one embodiment, the authentication information of the candidate health monitoring equipment to be connected contains the key set corresponding to the candidate health monitoring equipment to be connected, and the key set contains the keys and key identification corresponding to the candidate health monitoring equipment to be connected.

In one embodiment, before the associated information of the candidate health monitoring equipment to be connected transmitted from the base station is received, further comprising:
receiving the login request sent from the base station, wherein the login request contains the identification information and login key information of the base station;
verifying the identification information and login key information of the base station; returning the login token information to the base station if verification is passed, so that the base station will carry the login token when sending the data.

In a second aspect, the embodiment of the present application offers a data transmission device implemented at the server, comprising:
a first receiving unit, used to receive the health monitoring equipment activation success information, wherein the status of the health monitoring equipment is marked as "activated", and the health monitoring equipment activation success information is sent from the health monitoring equipment after the designated mobile terminal activates the health monitoring equipment or sent after the base station activates the health monitoring equipment;
a second receiving unit, used to receive the associated information of the candidate health monitoring equipment to be connected, wherein the candidate health monitoring equipment to be connected is the connectable health monitoring equipment to be connected detected by the base station within its coverage;
a return unit, used to return a message that instructs connection of the candidate health monitoring equipment to be connected to the base station if the connection conditions are determined to be met based on the associated information of the candidate health monitoring equipment to be connected and the status of the candidate health monitoring equipment to be connected, so that the base station will be connected to the candidate health monitoring equipment to be connected; and
a third receiving unit, used to receive the health monitoring data that is transmitted from the base station and collected from the health monitoring equipment connected to the base station.

In one embodiment, the associated information of the candidate health monitoring equipment to be connected includes the identification information of the candidate health monitoring equipment to be connected and the signal strength between the candidate health monitoring equipment to be connected and the base station;
wherein the return unit is specifically used to search for the status of candidate health monitoring equipment to be connected based on the identification of the candidate health monitoring equipment to be connected; if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the first base station at the moment the equipment was disconnected from the first base station to which it was connected last time, the connection conditions are confirmed to be met.

In one embodiment, the associated information of the candidate health monitoring equipment to be connected includes the identification information of the candidate health monitoring equipment to be connected and the signal strength between the candidate health monitoring equipment to be connected and the base station;
wherein the return unit is specifically used to search for the status of candidate health monitoring equipment to be connected based on the identification of the candidate health monitoring equipment to be connected; if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the second base station adjacent to the base station, the connection conditions are confirmed to be met.

In one embodiment, the message that instructs the connection of the candidate health monitoring equipment contains the authentication information of the candidate health monitoring equipment to be connected, so that the base station and the candidate health monitoring equipment to be connected will execute bidirectional authentication based on the authentication information, and they will connect after successful authentication.

In one embodiment, the authentication information of the candidate health monitoring equipment to be connected contains the key set corresponding to the candidate health monitoring equipment to be connected, and the key set contains the keys and key identification corresponding to the candidate health monitoring equipment to be connected.

In one embodiment, the device further comprises:
a fourth receiving unit, used to receive the login request sent from the base station before the associated information of candidate health monitoring equipment to be connected is received from the base station, wherein the login request contains the identification information and login key information of the base station; and
a verification unit, used to verify the identification information and login key information of the base station, and if verification is passed, return the login token information to the base station, so that the base station will carry the login token when sending the data.

In a third aspect, the embodiment of the present application also offers a data transmission method implemented at the base station, comprising:
sending the health monitoring equipment activation success information to the server after the to-be-activated health monitoring equipment is activated, so that the server will mark the status of the health monitoring equipment as "activated";
sending the associated information of the connectable candidate health monitoring equipment to be connected detected within the coverage to the server, so that the server will determine whether the connection conditions are met based on the associated information of the candidate health monitoring equipment to be connected and the status of the candidate health monitoring equipment to be connected;
connecting the candidate health monitoring equipment to be connected if the message that instructs the connection of the candidate health monitoring equipment to be connected is received from the server; and
receiving the health monitoring data reported from the connected health monitoring equipment, and sending the health monitoring data reported from the health monitoring equipment to the server.

In one embodiment, the to-be-activated health monitoring equipment will be activated in the following ways:
receiving the Bluetooth broadcast data packet sent from the to-be-activated health monitoring equipment, wherein the Bluetooth broadcast data packet is sent after the to-be-activated health monitoring equipment is woken up by the designated mobile terminal;
obtaining the identification information of the to-be-activated health monitoring equipment from the to-be-activated health monitoring equipment;
sending the identification information of the to-be-activated health monitoring equipment to the server, so that the server will verify the identification information of the to-be-activated health monitoring equipment;
receiving the authentication information of the to-be-activated health monitoring equipment sent from the server, wherein the authentication information of the to-be-activated health monitoring equipment is sent after the server verifies the identification information of the to-be-activated health monitoring equipment; and
executing bidirectional authentication with the to-be-activated health monitoring equipment based on the authentication information of the to-be-activated health monitoring equipment, and activating the to-be-activated health monitoring equipment after authentication is successful.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
executing bidirectional authentication with the to-be-activated health monitoring equipment based on the authentication information of the to-be-activated health monitoring equipment, specifically comprising:
generating the first public key and first private key according to the preset key negotiation algorithm;
selecting any first key from the key set, and generating the first signature information by using the Hash algorithm according to the selected first key, first time information and the first public key;
sending the first authentication request to the to-be-activated health monitoring equipment, wherein the first authentication request contains the identification information of the first key, the first time information, the first public key and the first signature information, so that the to-be-activated health monitoring equipment will generate the second signature information by using the Hash algorithm according to the key corresponding to the identification of the first key, the first time information and the first public key, so as to verify whether the second signature information matches the first signature information;
receiving the second authentication request sent when the to-be-activated health monitoring equipment confirms the second signature information matches the first signature information, wherein the second authentication request contains the identification information of the second key, second time information, second public key and third signature information, the third signature information is generated by the to-be-activated health monitoring equipment by using the Hash algorithm according to the selected second key, the second time information and the second public key and the second public key is generated by the to-be-activated health monitoring equipment by using the preset key negotiation algorithm;
generating the fourth signature information by using the Hash algorithm according to the key corresponding to the identification of the second key, the second time information and the second public key; and
confirming the authentication is successful if the fourth signature information is confirmed to match the third signature information.

In a fourth aspect, the embodiment of the present application also offers a data transmission device implemented at the base station, comprising:
a first transmitting unit, used to send the health monitoring equipment activation success information to the server after the to-be-activated health monitoring equipment is activated, so that the server will mark the status of the health monitoring equipment as "activated";
a second transmitting unit, used to send the associated information of the connectable candidate health monitoring equipment to be connected detected within the coverage to the server, so that the server will determine whether the connection conditions are met based on the associated information of the candidate health monitoring equipment to be connected and the status of the candidate health monitoring equipment to be connected;
a processing unit, used to connect the candidate health monitoring equipment to be connected if the message that instructs the connection of the candidate health monitoring equipment to be connected is received from the server; and
a third transmitting unit, used to receive the health monitoring data reported from the connected health monitoring equipment, and send the health monitoring data reported from the health monitoring equipment to the server.

In one embodiment, the device further comprises:
an activation unit, used to activate the to-be-activated health monitoring equipment by means of: receiving the Bluetooth broadcast data packet sent from the to-be-activated health monitoring equipment, wherein the Bluetooth broadcast data packet being sent after the to-be-activated health monitoring equipment is woken up by the designated mobile terminal; obtaining the identification information of the to-be-activated health monitoring equipment from the to-be-activated health monitoring equipment; sending the identification information of the to-be-activated health monitoring equipment to the server, so that the server will verify the identification information of the to-be-activated health monitoring equipment; receiving the authentication information of the to-be-activated health monitoring equipment sent from the server, wherein the authentication information of the to-be-activated health monitoring equipment sent from the server is sent after the server verifies the identification information of the to-be-activated health monitoring equipment; executing bidirectional authentication with the to-be-activated health monitoring equipment based on the authentication information of the to-be-activated health monitoring equipment, and activating the to-be-activated health monitoring equipment after authentication is successful.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
the activation unit is specifically used to generate the first public key and the first private key according to the preset key negotiation algorithm; select any first private key from the key set, and generate the first signature information by using the Hash algorithm according to the selected first private key, first time information and the first public key; send the first authentication request to the to-be-activated health monitoring equipment, wherein the first authentication request contains the identification information of the first key, the first time information, the first public key and the first signature information, so that the to-be-activated health monitoring equipment will generate the second signature information by using the Hash algorithm according to the key corresponding to the identification of the first key, the first time information and the first public key, so as to verify whether the second signature information matches the first signature information; receive the second authentication request that is sent when the to-be-activated health monitoring equipment confirms the second signature information matches the first signature information, wherein the second authentication request contains the identification information of the second key, second time information, second public key and third signature information, the third signature information is generated by the to-be-activated health monitoring equipment by using the Hash algorithm according to the selected second key, the second time information and the second public key, and the second public key is generated by the to-be-activated health monitoring equipment by using the preset key negotiation algorithm; generate the fourth signature information by using the Hash algorithm according to the key corresponding to the identification of the second key, the second time information and the second public key; confirm authentication is successful if the fourth signature information is confirmed to match the third signature information.

In a fifth aspect, the embodiment of the present application offers a data transmission method implemented at the designated mobile terminal, comprising:
waking up the to-be-activated health monitoring equipment, and obtaining the identification information of the to-be-activated health monitoring equipment;
sending the identification information of the to-be-activated health monitoring equipment to the server, so that the server will verify the identification of the to-be-activated health monitoring equipment;
receiving the authentication information of the to-be-activated health monitoring equipment sent from the server, wherein the authentication information of the to-be-activated health monitoring equipment is sent after the server verifies the identification of the to-be-activated health monitoring equipment;
executing bidirectional authentication with the to-be-activated health monitoring equipment based on the authentication information of the to-be-activated health monitoring equipment, wherein, after authentication is successful, the to-be-activated health monitoring equipment will be connected and the to-be-activated health monitoring equipment will be activated; and
sending the health monitoring equipment activation success information to the server, so that the server will mark the status of the health monitoring equipment as "activated"; disconnecting from the health monitoring equipment.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
executing bidirectional authentication with the to-be-activated health monitoring equipment based on the authentication information of the to-be-activated health monitoring equipment, specifically comprising:
generating the third public key and third private key according to the preset key negotiation algorithm;
selecting any third key from the key set, and generating the fifth signature information by using the Hash algorithm according to the selected third key, third time information and the third public key;
sending the third authentication request to the to-be-activated health monitoring equipment, wherein the third authentication request contains the identification information of the third key, the third time information, the third public key and the fifth signature information, so that the to-be-activated health monitoring equipment will generate the sixth signature information by using the Hash algorithm according to the key corresponding to the identification of the third key, the third time information and the third public key, so as to verify whether the sixth signature information matches the fifth signature information;
receiving the fourth authentication request sent when the to-be-activated health monitoring equipment confirms the sixth signature information matches the fifth signature information, wherein the fourth authentication request contains the identification information of the fourth key, fourth time information, fourth public key and seventh signature information, the seventh signature information is generated by the to-be-activated health monitoring equipment by using the Hash algorithm according to the selected fourth key, the fourth time information and the fourth public key, and the fourth public key is generated by the to-be-activated health monitoring equipment according to the preset key negotiation algorithm;
generating the eighth signature information by using the Hash algorithm according to the key corresponding to the identification of the fourth key, the fourth time information and the fourth public key; and
confirming the authentication is successful if the eighth signature information is confirmed to match the seventh signature information.

In one embodiment, before the identification information of the to-be-activated health monitoring equipment is sent to the server, further comprising:
sending a login request to the server, wherein the login request contains the login information; and
receiving the access token information returned from the server, wherein the access token is generated by the server after the login information is successfully verified.

In a sixth aspect, the embodiment of the present application offers a data transmission device implemented at the designated mobile terminal, comprising:
a wake-up unit, used to wake up the to-be-activated health monitoring equipment, and access the identification information of the to-be-activated health monitoring equipment;
a first transmitting unit, used to send the identification information of the to-be-activated health monitoring equipment to the server, so that the server will verify the identification of the to-be-activated health monitoring equipment;
a first receiving unit, used to receive the authentication information of the to-be-activated health monitoring equipment sent from the server, wherein the authentication information of the to-be-activated health monitoring equipment is sent after the server verifies the identification of the to-be-activated health monitoring equipment;
an authentication unit, used to execute bidirectional authentication with the to-be-activated health monitoring equipment based on the authentication information of the to-be-activated health monitoring equipment, and connect the to-be-activated health monitoring equipment and activate the to-be-activated health monitoring equipment after authentication is successful; and
a second transmitting unit, used to send the health monitoring equipment activation success information to the server, so that the server will mark the status of the health monitoring equipment as "activated"; disconnect from the health monitoring equipment.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
the authentication unit is specially used to generate the third public key and third private key according to the preset key negotiation algorithm; select any third key from the key set, and generate the fifth signature information by using the Hash algorithm according to the third key selected, the third time information and the third public key; send the third authentication request to the to-be-activated health monitoring equipment, wherein the third authentication request contains the identification information of the third key, the third time information, the third public key and the fifth signature information, so that the to-be-activated health monitoring equipment will generate the sixth signature information by using the Hash algorithm according to the key corresponding to the identification of the third key, the third time information and the third public key, so as to verify whether the sixth signature information matches the fifth signature information; receive the fourth authentication request that is sent when the to-be-activated health monitoring equipment confirms the sixth signature information matches the fifth signature information, wherein the fourth authentication request contains the identification information of the fourth key, fourth time information, fourth public key and seventh signature information, the seventh signature information is generated by the to-be-activated health monitoring equipment by using the Hash algorithm according to the selected fourth key, the fourth time information and the fourth public key, and the fourth public key is generated by the to-be-activated health monitoring equipment by using the preset key negotiation algorithm; generate the eighth signature information by using the Hash algorithm according to the key corresponding to the identification of the fourth key, the fourth time information and the fourth public key; confirm authentication is successful if the eighth signature information is confirmed to match the seventh signature information.

In one embodiment, the device further comprises:
a third transmitting unit, used to send the login request to the server before the identification information of the to-be-activated health monitoring equipment is sent to the server, wherein the login request contains the login information;
a second receiving unit, used to receive the access token information returned from the server, wherein the access token is generated by the server after the login information is successfully verified.

In a seventh aspect, the embodiment of the present application offers a data transmission method implemented at the health monitoring equipment, comprising:
receiving the request for access to identification information of health monitoring equipment sent from the designated mobile terminal or base station, wherein the request for access to identification information of health monitoring equipment is sent from the designated mobile terminal or base station after the health monitoring equipment is woken up by the designated mobile terminal;
returning the identification information of the health monitoring equipment to the designated mobile terminal or the base station, so that the designated mobile terminal or the base station will activate the health monitoring equipment according to the identification information of the health monitoring equipment;
sending the associated information of the health monitoring equipment to the connectable base station after activation, so that the base station will send the associated information of the health monitoring equipment to the server, and the server will determine whether the connection conditions are met based on the associated information of the health monitoring equipment and the status of the health monitoring equipment; and
reporting the collected health monitoring data to the base station after connecting the base station, so that the base station will send the health monitoring data to the server, wherein the health monitoring equipment is connected to the base station after the base station receives the message that instructs the connection of the

In one embodiment, the message that instructs the connection of the health monitoring equipment contains the authentication information of the health monitoring equipment;
before connection with the base station, the method further comprises:
executing bidirectional authentication with the base station based on the authentication information of the health monitoring equipment; and
confirming bidirectional authentication is successful.

In one embodiment, executing bidirectional authentication with the base station based on the authentication information of the health monitoring equipment, specifically comprising:
receiving the fifth authentication request sent from the base station, wherein the fifth authentication request contains the identification information of the fifth key, fifth time information, fifth public key and ninth signature information, the fifth public key is generated by the base station according to the preset key negotiation algorithm, the fifth key is selected by the base station from the key set contained in the authentication information of the health monitoring equipment returned from the server, and the ninth signature information is generated by the base station by using the Hash algorithm according to the fifth key, the fifth event information and the fifth public key;
generating the tenth signature information by using the Hash algorithm according to the key corresponding to the identification of the fifth key, the fifth time information and the fifth public key;
generating the sixth public key and sixth private key according to the preset key negotiation algorithm if the tenth signature information is confirmed to match the ninth signature information;
selecting any sixth key from the locally stored key set, and generating the eleventh signature information by using the Hash algorithm according to the selected sixth key, sixth time information and the sixth public key;
sending the sixth authentication request to the base station, wherein the sixth authentication request contains the identification information of the sixth key, the sixth time information, the sixth public key and the eleventh signature information, so that the base station will generate the twelfth signature information by using the Hash algorithm according to the key corresponding to the identification of the sixth key, the sixth time information and the sixth public key, so as to verify whether the twelfth signature information matches the eleventh signature information.

In one embodiment, if bidirectional authentication is successful, the method further comprises:
co-generating the shared key with the base station by using the preset key negotiation algorithm based on the fifth public key, the fifth private key corresponding to the fifth public key, the sixth public key and the sixth private key;
sending the shared key to the server, so that the server will store the correspondence between the identification information of the health monitoring equipment and the shared key; and
reporting the collected health monitoring data to the base station, so that the base station will send the health monitoring data to the server, specifically comprising:
   encrypting the health monitoring data according to the shared key and preset encryption algorithm, to generate the encrypted health monitoring data;
   sending the encrypted health monitoring data to the server, so that the server will decrypt the encrypted health monitoring data according to the shared key.

In an eighth aspect, the embodiment of the present application offers a data transmission device implemented at the health monitoring equipment, comprising:
a receiving unit, used to receive the request for access to identification information of health monitoring equipment sent from the designated mobile terminal or base station, wherein the request for access to identification information of health monitoring equipment is sent from the designated mobile terminal or base station after the health monitoring equipment is woken up by the designated mobile terminal;
a return unit, used to return the identification information of the health monitoring equipment to the designated mobile terminal or the base station, so that the designated mobile terminal or the base station will activate the health monitoring equipment according to the identification information of the health monitoring equipment;
a first transmitting unit, used to send the associated information of the health monitoring equipment to the connectable base station after activation, so that the base station will send the associated information of the health monitoring equipment to the server, wherein the server will determine whether the connection conditions are met based on the associated information of the health monitoring equipment and the status of the health monitoring equipment;
a reporting unit, used to report the collected health monitoring data to the base station after the base station is connected, so that the base station will send the health monitoring data to the server, wherein the health monitoring equipment is connected to the base station after the base station receives the message that instructs the connection of the health monitoring equipment returned from the server.

In one embodiment, the message that instructs the connection of the health monitoring equipment contains the authentication information of the health monitoring equipment;
the device further comprising:
an authentication unit, used to execute bidirectional authentication with the base station based on the authentication information of the health monitoring equipment before the base station is connected;
a determination unit, used to determine bidirectional authentication is successful.

In one embodiment, the authentication unit is specifically used to receive the fifth authentication request sent from the base station, wherein the fifth authentication request contains the identification information of the fifth key, fifth time information, fifth public key and ninth signature information, the fifth public key is generated by the base station according to the preset key negotiation algorithm, the fifth key is selected by the base station from the key set contained in the authentication information of the health monitoring equipment returned from the server, and the ninth signature information is generated by the base station by using the Hash algorithm according to the fifth key, the fifth event information and the fifth public key; generate the tenth signature information by using the Hash algorithm according to the key corresponding to the fifth key, the fifth time information and the fifth public key; generate the sixth public key and sixth private key according to the preset key negotiation algorithm if the tenth signature information is confirmed to match the ninth signature information; selecting any sixth key from the locally stored key set, and generate the eleventh signature information by using the Hash algorithm according to the selected sixth key, sixth time information and sixth public key; send the sixth authentication request to the base station, wherein the sixth authentication request contains the identification information of the sixth key, the sixth time information, the sixth public key and the eleventh signature information, so that the base station will generate the twelfth signature information by using the Hash algorithm according to the key corresponding to the identification of the sixth key, the sixth time information and the sixth public key, so as to verify whether the twelfth signature information matches the eleventh signature information.

In one embodiment, further comprising:
a key negotiation unit, used to co-generate the shared key with the base station by using the preset key negotiation algorithm based on the fifth public key, the fifth private key corresponding to the fifth public key, the sixth public key and the sixth private key if bidirectional authentication is confirmed to be successful;
a second transmitting unit, used to send the shared key to the server, so that the server will store the correspondence between the identification information of the health monitoring equipment and the shared key; and
a reporting unit, specifically used to encrypt the health monitoring data according to the shared key and preset encryption algorithm, so as to generate encrypted health monitoring data; sending the encrypted health monitoring data to the server, so that the server will decrypt the encrypted health monitoring data according to the shared key.

In a ninth aspect, the embodiment of the present application offers an electronic equipment comprising memory, processor and the computer program that is stored on the memory and operable on the processor, wherein the processor executes the program to implement the data transmission method described in the present application.

In a tenth aspect, the embodiment of the present application offers a computer-readable storage medium, in which the computer program is stored, wherein the program is executed by the processor to implement the steps of the data transmission method described in the present application.

The beneficial effects of the present application are as follows:
the data transmission method, device, electronic equipment, and storage medium provided in embodiment of the present application activates the health monitoring equipment via the designated mobile terminal or base station; after the health monitoring equipment is activated successfully and the health monitoring equipment activation success information is sent to the server, the server marks the status of the health monitoring equipment as "activated"; the base station detects the connectable health monitoring equipment to be connected within its coverage, designates the health monitoring equipment to be connected as candidate health monitoring equipment to be connected, and sends the associated information of the candidate health monitoring equipment to be connected to the server; if the server determines the connection conditions are met based on the associated information of the candidate health monitoring equipment to be connected and the status of the candidate health monitoring equipment to be connected, it will return a message that instructs the connection of the candidate health monitoring equipment to be connected to the base station; the base station will be connected to the candidate health monitoring equipment to be connected and will send the health monitoring data collected from the connected health monitoring equipment to the server; in the embodiment of the present application, the mobile terminal of the monitored object is not needed to activate health monitoring equipment or upload the health monitoring data connected by the health monitoring equipment to the server; instead, a designated mobile terminal (e.g. the mobile terminal of a medical worker or a separate mobile terminal) or the base station is used to activate the health monitoring equipment, and the base station will detect the connectable candidate health monitoring equipment to be connected within its coverage; the server will determine whether the candidate health monitoring equipment to be connected meets the connection conditions; the base station will be connected to the candidate health monitoring equipment to be connected that meet the connection conditions; the base station can in real time upload the health monitoring data collected from the health monitoring equipment to the server; this will solve the problem where the health monitoring equipment cannot be activated and the health monitoring data cannot be uploaded because the monitored object does not have a mobile phone or any other mobile terminal or does not know how to use the health monitoring application program in the mobile terminal; besides, the base station provided in embodiment of the present application will in real time detect the connectable health monitoring equipment within its coverage; if the monitored object wearing the health monitoring equipment moves and such movement results in communication disconnection between the health monitoring equipment and the connected base station, the equipment will be immediately detected by the base station whose coverage reaches the health monitoring equipment, so the equipment will be connected to the base station to continue uploading the health monitoring data in real time.

The other characteristics and advantages of the present application will be explained in the subsequent description and will partly become comprehensible through the description or become understandable through implementation of the present application. The purpose and other advantages of the present application can be achieved and obtained through the structure specifically explained by the Description, Claims and Drawings.

### Brief Description of the Drawings

The drawings provided here are used for further description of the present application, which are an integral part of the present application. Illustrative embodiments of the present application and the descriptions thereof shall not pose improper limitation to the present application. In the drawings:
FIG. 1 illustrates a schematic diagram for the application scenario of the data transmission method as provided in the embodiment of the present application;
FIG. 2 illustrates a schematic diagram for the implementation process of activating the health monitoring equipment via the base station as provided in the embodiment of the present application;
FIG. 3 illustrates a schematic diagram for the implementation process of bidirectional authentication between the base station and to-be-activated health monitoring equipment as provided in the embodiment of the present application;
FIG. 4 illustrates a schematic diagram for the implementation process of activating the health monitoring equipment via the designated mobile terminal as provided in the embodiment of the present application;
FIG. 5 illustrates a schematic diagram for the implementation process of bidirectional authentication between designated mobile terminal and to-be-activated health monitoring equipment as provided in the embodiment of the present application;
FIG. 6 illustrates a schematic diagram for a disconnection scenario of the health monitoring equipment as provided in the embodiment of the present application;
FIG. 7 illustrates a schematic diagram for the data transmission method as provided in the embodiment of the present application;
FIG. 8 illustrates a schematic diagram for the implementation process of bidirectional authentication between the base station and candidate health monitoring equipment to be connected as provided in the embodiment of the present application;
FIG. 9 illustrates a schematic diagram for the implementation process of the data transmission method implemented at the server as provided in the embodiment of the present application;
FIG. 10 illustrates a structural schematic diagram for the data transmission device implemented at the server as provided in the embodiment of the present application;
FIG. 11 illustrates a schematic diagram for the implementation process of the data transmission method implemented at the base station as provided in the embodiment of the present application;
FIG. 12 illustrates a structural schematic diagram for the data transmission device implemented at the base station as provided in the embodiment of the present application;
FIG. 13 illustrates a schematic diagram for the implementation process of the data transmission method implemented at the designated mobile terminal as provided in the embodiment of the present application;
FIG. 14 illustrates a structural schematic diagram for the data transmission device implemented at the designated mobile terminal as provided in the embodiment of the present application;
FIG. 15 illustrates a schematic diagram for the implementation process of the data transmission method implemented at the health monitoring equipment as provided in the embodiment of the present application;
FIG. 16 illustrates a structural schematic diagram for the data transmission device implemented at the health monitoring equipment as provided in the embodiment of the present application;
FIG. 17 illustrates a structural schematic diagram for the electronic equipment as provided in embodiment of the present application.

### Detailed Description of the Preferred Embodiments

In order to solve the problems that activation of health monitoring equipment and uploading of health monitoring data may fail due to the limitations of the prior art and that the communication disconnection between the mobile terminal of the monitored object and the health monitoring equipment may cause the health monitoring data of the monitored object to fail to be uploaded continuously in real time, the embodiment of the present application offers a data transmission method, device, electronic equipment, and storage medium.

The preferred embodiments of the present application are described below in conjunction with the drawings in the description. It should be understood that the preferred embodiments described herein are only used to illustrate and explain the present application rather than defining the present application. The embodiments in the present application and the characteristics of the embodiments can be combined as long as they are not conflicted.

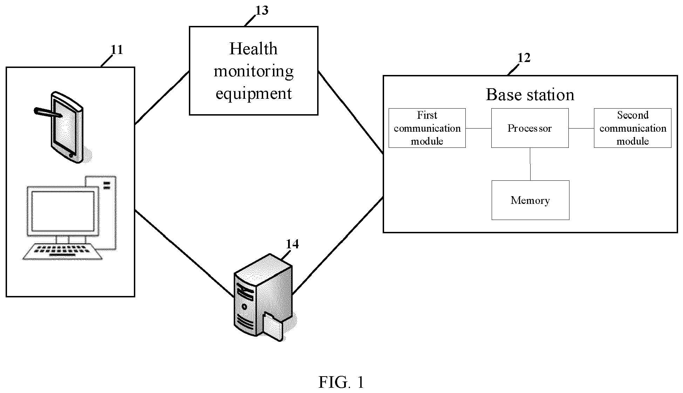

First see FIG. 1, which illustrates a schematic diagram for one application scenario for the data transmission method as provided in embodiment of the present application, wherein the scenario may include designated mobile terminal 11, base station 12, health monitoring equipment 13 and server 14. The designated mobile terminal 11 can be a health monitoring of a medical worker or an independent mobile terminal, and the designated mobile terminal 11 is used to wake up health monitoring equipment 13 or wake up and activate health monitoring equipment 13. Base station 12 can be set by the user as needed in a place such as hospital or nursing home, e.g. a base station 12 can be installed every several rooms, wherein the embodiment of the present application does not define a limit to this; unlike the base station in the prior art, the base station 12 provided in the embodiment of the present application can be used to wake up the health monitoring equipment 13 at the designated mobile terminal 11 and then activate health monitoring equipment 13, detect the health monitoring equipment 13 within its coverage, connect the health monitoring equipment 13 that meets the connection conditions, collect the health monitoring data of the monitored object being monitored by health monitoring equipment 13, and upload the health monitoring data to server 14; base station 12 can also be used to wake up and activate health monitoring equipment 13 at designated mobile terminal 11, and then detect the health monitoring equipment 13 within its coverage, connect the health monitoring equipment 13 that meets the connection conditions, collect the health monitoring data of the monitored object being monitored by health monitoring equipment, and upload the health monitoring data to server 14. The base station 13 provided in the embodiment of the present application is equivalent to a data relay forwarding device; base station 13 can include the following modules: first communication module, processor, second communication module and memory; the first communication module, second communication module and memory are connected to the processor; the first communication module can be a Bluetooth communication module; the second communication module can be a Wi-Fi communication module or a 4G (4th-Generation Mobile Communication Technology) or 5G (5th-Generation Mobile Communication Technology) communication module; the embodiment of the present application does not define a limit to this. Base station 12 is connected to health monitoring equipment 13 via the first communication module; the first communication module can be connected to multiple health monitoring equipment 13s simultaneously; the number of health monitoring equipment 13s connected to base station 12 can be preset; for example, the number can be preset as but not limited to 10; the embodiment of the present application does not define a limit to this; base station 12 is connected to server 14 via the second communication module; if the second communication module is a Wi-Fi communication module, this Wi-Fi communication module can support the communication and data transmission between base station 12 and server 14 through Wi-Fi connection; if the second communication module is a 4G or 5G communication module, the 4G or 5G communication module can have built-in ESIM (embedded subscriber identity module) card to support the communication and data transmission between base station 12 and server 14; the processor is used to complete the whole process control and task scheduling; the memory is used to store authentication information of health monitoring equipment 13 and the connection information of health monitoring equipment, and to cache health monitoring data reported by the health monitoring equipment.

In the embodiment of the present application, designated mobile terminal 11 can be but not limited to: smartphone, tablet computer, PDA (Personal Digital Assistant, a portable electronic equipment), laptop and desktop computer; server 14 can be an independent physical server or a cloud server that provides the basic cloud computing services such as cloud server, cloud database and cloud storage; the embodiment of the present application does not define a limit to this.

Set in the above application scenarios, the exemplary embodiments are described in greater detail below by using drawings 2-4; it must be noted that the above application scenarios are only intended to help the reader understand the rationale and principle of the present application, and the embodiment of the present application is not limited in any way here. On the contrary, the embodiment of the present application is applicable in any scenario.

The data transmission method provided in the embodiment of the present application can include two stages: activation of health monitoring equipment and uploading of health monitoring data.

When the health monitoring equipment is used for the first time, it needs to be activated. In one embodiment, the health monitoring equipment can be woken up by the designated mobile terminal and then it can be activated by the base station; then the health monitoring data reported by the health monitoring equipment will be uploaded to the server by the base station.

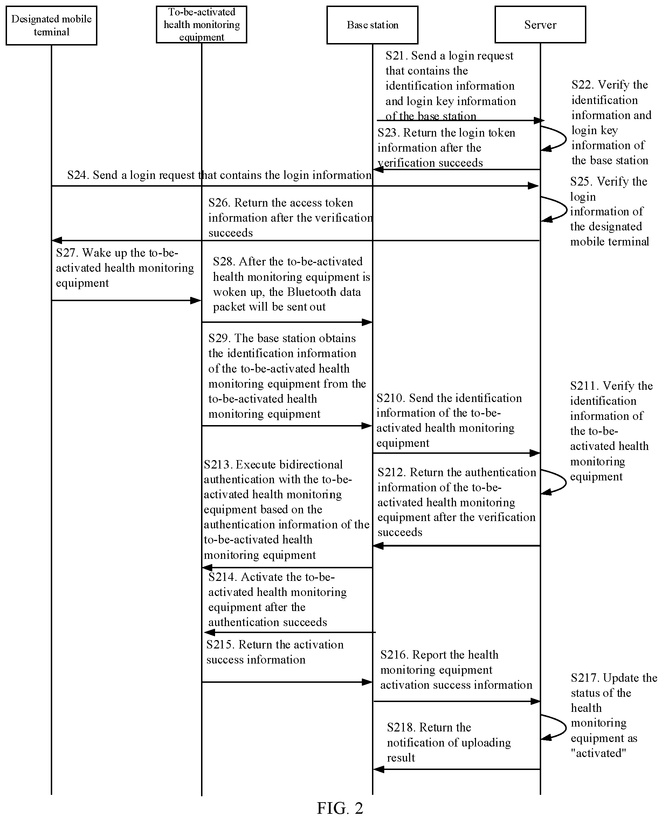

The implementation process of activating the health monitoring equipment by the base station is shown in FIG. 2; this stage involves the interaction between the designated mobile terminal, health monitoring equipment, base station and server, and can include the following steps:
S21. The base station sends a login request to the server, wherein the login request contains the identification information and login key information of the base station.

In a specific implementation, the base station delivered from the manufacturer carries the identification and login key that are preset by the manufacturer, wherein the identification is unique to the base station, the login key is used for the identity authentication executed by the server when the base station logs in to the server, and the server already stores the correspondence between the identification and login key of the base station. When the base station logs in to the server for the first time, the base station sends a login request that contains its identification information and login key information to the server.

S22. The server verifies the identification information and login key information of the base station; if verification is passed, Step S23 is executed; if not, the message of login failure will be returned to the base station.

In a specific implementation, the server will verify the identification and login key of the base station carried in the login request sent from the base station according to the correspondence between the identification and login key of the base station; if the login key in the login request matches the login key corresponding to the identification in the correspondence between the identification and login key of the station already stored, verification is passed and the base station logs in to the server successfully.

S23. The server returns login token information to base station.

In a specific implementation, if the server's verification of the identification and login key of base station is passed, the server will generate the login token for the base station, store the correspondence between the identification and login token of the base station, and return the login token information to the base station; when the base station sends data to the server later, the data will carry the login token information; the server will search for the login token corresponding to the base station from the stored correspondence between the identification and access token of the base station, so as to verify the login token sent from the base station; if the two tokens match, verification is passed; this will enhance safety of data transmission between base station and server.

S24. The designated mobile terminal sends a login request to the server, wherein the login request contains the login information.

The login information can include user name and password, and can also include mobile phone number and verification code, and the embodiment of the present application does not define a limit to this.

S25. The server verifies the login information of the designated mobile terminal; if verification is passed, Step S26 is executed; if not, the message of login failure will be returned to the designated mobile terminal.

The server already stores the login information of the registered mobile terminal: user name and password, or mobile phone number; if the login request received from the designated mobile terminal contains the user name and password, the user name and password will be compared against the stored user name and password of the mobile terminal; if they match, verification is passed; if the login request contains the mobile phone number of the registered user, a verification code will be sent to the mobile phone number; if the verification code in the received login request matches the one sent from the server, verification is passed, and the designated mobile terminal successfully logs in to the server.

S26. The server returns access token information to the designated mobile terminal.

In a specific implementation, if the server's verification of the login information of the designated mobile terminal is passed, the server will generate the access token for the designated mobile terminal, store the correspondence between the identification and access token of the designated mobile terminal, and return the access token information to the designated mobile terminal. When the designated mobile terminal sends data to the server later, the data can carry the access token, the server will search for the access token corresponding to the identification of the designated mobile terminal from the stored correspondence between the identification and access token of the mobile terminal, so as to verify the access token sent from the designated mobile terminal; if the two tokens match, verification is passed; this will enhance safety of data transmission between designated mobile terminal and server.

S27. The designated mobile terminal will wake up the to-be-activated health monitoring equipment.

In a specific implementation, the designated mobile terminal can wake up the to-be-activated health monitoring equipment through (but not limited to) near field communication.

S28. After the to-be-activated health monitoring equipment is woken up, the Bluetooth data packet will be sent out.

In this step, after the to-be-activated health monitoring equipment is woken up by the designated mobile terminal, it will continually send Bluetooth broadcast data packets in the preset time period, wherein the time period can be set by the user as needed, and the embodiment of the present application does not define a limit to this.

S29. The base station receives the Bluetooth data packet sent from the to-be-activated health monitoring equipment, and obtains the identification information of the to-be-activated health monitoring equipment from the to-be-activated health monitoring equipment.

In a specific implementation, the identification information of the health monitoring equipment can be the MAC address (Media Access Control Address, physical address) information used for Bluetooth interaction, and the base station receives the Bluetooth data packet sent from the to-be-activated health monitoring equipment and reads the MAC address information of the to-be-activated health monitoring equipment from the to-be-activated health monitoring equipment.

S210. The base station sends the identification information of the to-be-activated health monitoring equipment to the server.

In a specific implementation, the base station sends the MAC address information of the to-be-activated health monitoring equipment to the server.

S211. The server verifies the identification information of the to-be-activated health monitoring equipment; if such information passes the verification, Step S212 is executed; otherwise, the to-be-activated health monitoring equipment is judged to be invalid, and the verification failure message is returned to the base station.

In a specific implementation, the server already stores the identification information of the health monitoring equipment (i.e. MAC address information) and validity period information of the health monitoring equipment; when the server receives the MAC address information of the to-be-activated health monitoring equipment sent from the base station, the server will check whether the MAC address information of the to-be-activated health monitoring equipment is stored locally; if so, and the current time is within the validity period of the to-be-activated health monitoring equipment, verification is passed.

S212. The server returns authentication information of the to-be-activated health monitoring equipment to the base station.

In a specific implementation, each health monitoring equipment delivered from the manufacturer carries the key set that is preset by the manufacturer, wherein the key set contains multiple keys and the key identification of each key, and the server stores the key set of each health monitoring equipment and uses it as authentication information.

After the server's verification of the identification information of the to-be-activated health monitoring equipment is passed, the server will return the key set corresponding to the to-be-activated health monitoring equipment to the base station, wherein the key set contains the key and key identification corresponding to the to-be-activated health monitoring equipment.

S213. The base station will execute bidirectional authentication with the to-be-activated health monitoring equipment based on the authentication information of the to-be-activated health monitoring equipment; if the authentication succeeds, Step S214 is executed; if the authentication fails, the message of authentication failure is returned to the server.

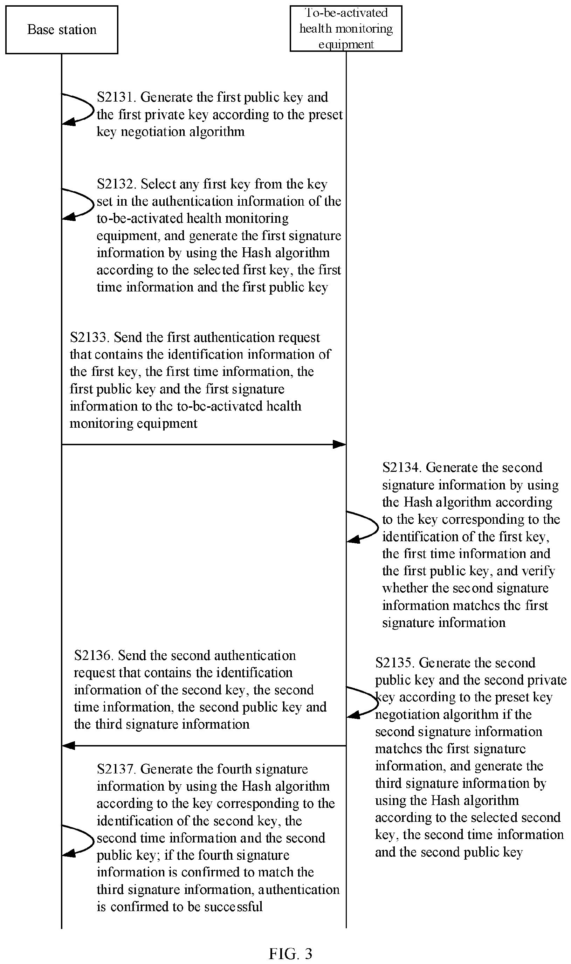

In a specific implementation, the base station and to-be-activated health monitoring equipment will execute bidirectional authentication in the process shown in FIG. 3, wherein the following steps can be executed:
S2131. The base station generates the first public key and first private key according to the preset key negotiation algorithm.

In a specific implementation, the base station can generate a public key and the corresponding private key according to the ECDH (elliptic curve Diffie-Hellman key exchange) key negotiation algorithm, and they can be identified as the first public key and first private key, wherein the first public key and first private key can also be generated by using any other key negotiation algorithm and the embodiment of the present application does not define a limit to this; in the embodiment of the present application, the ECDH key negotiation algorithm is only used as an example.

S2132. The base station selects any first key from the key set in the authentication information of the to-be-activated health monitoring equipment, and generates the first signature information by using the Hash algorithm according to the selected first key, first time information and first public key.

In a specific implementation, after the base station receives the authentication information of the to-be-activated health monitoring equipment returned from the server, the server will select any key from the key set of the to-be-activated health monitoring equipment contained in the authentication information of the to-be-activated health monitoring equipment, and this key can be identified as the first key; the signature information is generated by using the Hash algorithm according to the first key, first time information and first public key; the signature information can be identified as first signature information; the first signature information can be the current time information.

S2133. The base station sends the first authentication request to the to-be-activated health monitoring equipment, wherein the first authentication request contains the identification information of the first key, the first time information, first public key and first signature information.

S2134. The to-be-activated health monitoring equipment generates the second signature information by using the Hash algorithm according to the key corresponding to the identification of the first key, the first time information and first public key, and verifies whether the second signature information matches the first signature information; if the second signature information matches the first signature information, Step S2135 is executed; if not, verification fails, and the message of verification failure is returned to the base station.

In a specific implementation, the to-be-activated health monitoring equipment will search for the key corresponding to the identification of the first key from the locally stored key set according to the identification of the first key sent from the base station, generate the second signature information by using the Hash algorithm according to the found key, the first time information sent from the base station and the first public key, and verify whether the second signature information matches the first signature information sent from the base station; if the second signature information is confirmed to match the first signature information, unidirectional authentication is confirmed to be successful; if the second signature information does not match the first signature information, authentication is confirmed to fail, the message of authentication failure is returned to the base station, and the base station will report the message of to-be-activated health monitoring equipment authentication failure to the server.

S2135. The to-be-activated health monitoring equipment will generate the second public key and second private key according to the preset key negotiation algorithm, and generate the third signature information by using the Hash algorithm according to the selected second key, second time information and second public key.

In a specific implementation, the to-be-activated health monitoring equipment will generate a public key and the corresponding private key according to the ECDH key negotiation algorithm, and the two keys are marked as second public key and second private key; the to-be-activated health monitoring equipment selects any key from the locally stored key set, and this key can be marked as the second key; the third signature information is generated by using the Hash algorithm according to the selected second key, second time information and second public key; the second time information can be the current time information.

S2136. The to-be-activated health monitoring equipment sends the second authentication request to the base station, wherein the second authentication request contains the identification information of the second key, the second time information, second public key and third signature information.

S2137. The base station generates the fourth signature information by using the Hash algorithm according to the key corresponding to the identification of the second key, the second time information and second public key; if the fourth signature information is confirmed to match the third signature information, authentication is confirmed to be successful.

In a specific implementation, the base station will search for the key corresponding to the identification of the second key from the key set of the to-be-activated health monitoring equipment contained in the authentication information of the to-be-activated health monitoring equipment, generate the fourth signature information by using the Hash algorithm according to the key corresponding to the identification of the found second key, the second time information sent from the base station and the second public key, and verify whether the fourth signature information matches the third signature information sent from the to-be-activated health monitoring equipment; if the fourth signature information is confirmed to match the third signature information, the bidirectional authentication between base station and to-be-activated health monitoring equipment is confirmed to be successful; if the fourth signature information does not match the third signature information, authentication is confirmed to fail, and the message of to-be-activated health monitoring equipment authentication failure is returned the server.

S214. The base station activates the to-be-activated health monitoring equipment.

In a specific implementation, after the bidirectional authentication between the base station and the to-be-activated health monitoring equipment succeeds, the base station will receive a Bluetooth data packet sent by the to-be-activated health monitoring equipment and establish a connection with the to-be-activated health monitoring equipment to activate the to-be-activated health monitoring equipment.

S215. The activated health monitoring equipment returns the activation success information to the base station.

In this step, the to-be-activated health monitoring equipment returns the activation success information to the base station after being activated.

S216. The base station reports the health monitoring equipment activation success information to the server.

In this step, the base station reports the activation success information of the activated health monitoring equipment to the server.

S217. The server updates the status of the health monitoring equipment as "activated".

In this step, the server updates the status of the activated health monitoring equipment as "updated" and saves the status.

In one embodiment, after waking up the to-be-activated health monitoring equipment, the designated mobile terminal can send the MAC address information of the to-be-activated health monitoring equipment to the server and subscribe the information of the to-be-activated health monitoring equipment; after receiving the health monitoring equipment activation success information, the server can push the health monitoring equipment activation success information to the designated mobile terminal, so that the designated mobile terminal will record the status of the health monitoring equipment and thus the medical worker can view the status of the health monitoring equipment via the designated mobile terminal.

S218. The server returns the notification of uploading result to the base station. Therefore, the activation process of health monitoring equipment is completed.

In one embodiment, the health monitoring equipment can be woken up by the designated mobile terminal; after the health monitoring equipment is activated, the health monitoring data reported from the health monitoring equipment will be uploaded to the server via the base station.

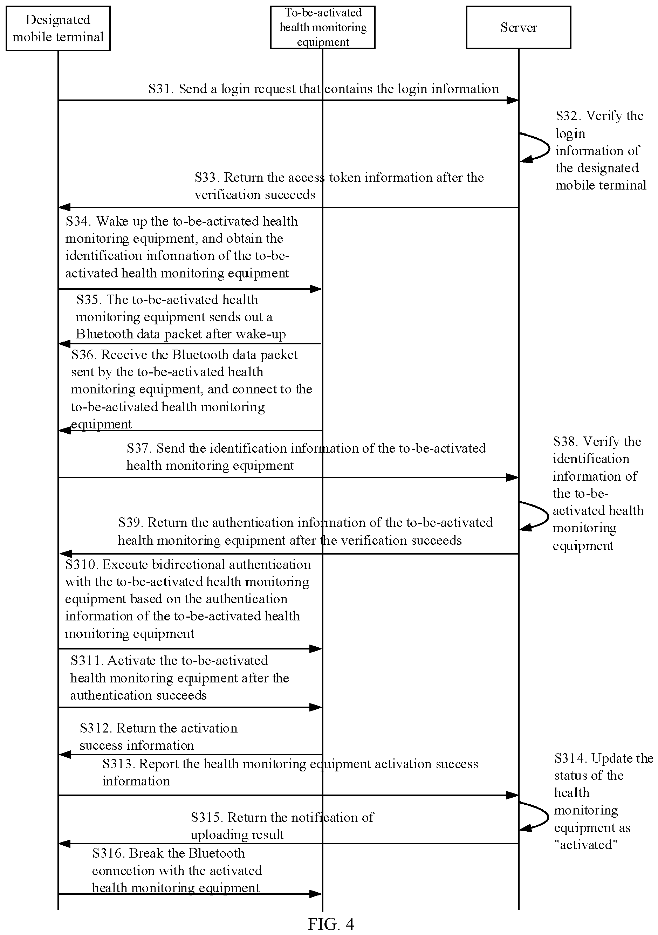

The implementation process of activating the health monitoring equipment by the mobile terminal is shown in FIG. 4; this stage involves the interaction between the designated mobile terminal, health monitoring equipment, and server, and can include the following steps:
S31. The designated mobile terminal sends login request to the server, wherein the login request contains the login information.

The login information can include user name and password, and can also include mobile phone number and verification code, and the embodiment of the present application does not define a limit to this.

S32. The server verifies the login information of the designated mobile terminal; if verification is passed, Step S33 is executed; if not, the message of login failure will be returned to the designated mobile terminal.

The server already stores the login information of the registered mobile terminal: user name and password, or mobile phone number; if the login request received from the designated mobile terminal contains the user name and password, the user name and password will be compared against the stored user name and password of the mobile terminal; if they match, verification is passed; if the login request contains the mobile phone number of the registered user, a verification code will be sent to the mobile phone number; if the verification code in the received login request matches the one sent from the server, verification is passed, and the designated mobile terminal successfully logs in to the server.

S33. The server returns access token information to the designated mobile terminal.

In a specific implementation, if the server's verification of the login information of the designated mobile terminal is passed, the server will generate the access token for the designated mobile terminal, store the correspondence between the identification and access token of the designated mobile terminal, and return the access token information to the designated mobile terminal. When the designated mobile terminal sends data to the server later, the data can carry the access token, the server will search for the access token corresponding to the identification of the designated mobile terminal from the stored correspondence between the identification and access token of the mobile terminal, so as to verify the access token sent from the designated mobile terminal; if the two tokens match, verification is passed; this will enhance safety of data transmission between designated mobile terminal and server.

S34. The designated mobile terminal wakes up the to-be-activated health monitoring equipment and obtains the identification information of the to-be-activated health monitoring equipment.

In a specific implementation, the designated mobile terminal may wake up the to-be-activated health monitoring equipment by means including but not limited to near field communication and may read the MAC address information of the health monitoring equipment from the to-be-activated health monitoring equipment.

S35. The to-be-activated health monitoring equipment sends out a Bluetooth data packet after wake-up.

In this step, after the to-be-activated health monitoring equipment is woken up by the designated mobile terminal, it will continually send Bluetooth broadcast data packets in the preset time period, wherein the time period can be set by the user as needed, and the embodiment of the present application does not define a limit to this.

S36. The designated mobile terminal receives the Bluetooth data packet sent by the to-be-activated health monitoring equipment and is connected to the to-be-activated health monitoring equipment.

In a specific implementation, the designated mobile terminal receives the Bluetooth data packet sent by the to-be-activated health monitoring equipment and establishes a Bluetooth connection with the to-be-activated health monitoring equipment.

S37. The designated mobile terminal sends the identification information of the to-be-activated health monitoring equipment to the server.

In a specific implementation, the designated mobile terminal sends the MAC address information of the to-be-activated health monitoring equipment to the server.

S38. The server verifies the identification information of the to-be-activated health monitoring equipment. If such information passes the verification, Step S39 is executed; otherwise, the to-be-activated health monitoring equipment is judged to be invalid, and the verification failure message is returned to the designated mobile terminal.

In a specific implementation, the server already stores the identification information of the health monitoring equipment (i.e. MAC address information) and validity period information of the health monitoring equipment; when the server receives the MAC address information of the to-be-activated health monitoring equipment sent from the base station, the server will check whether the MAC address information of the to-be-activated health monitoring equipment is stored locally; if so, and the current time is within the validity period of the to-be-activated health monitoring equipment, verification is passed.

S39. The server returns the authentication information of the to-be-activated health monitoring equipment to the designated mobile terminal.

In a specific implementation, each health monitoring equipment delivered from the manufacturer carries the key set that is preset by the manufacturer, wherein the key set contains multiple keys and the key identification of each key, and the server stores the key set of each health monitoring equipment and uses it as authentication information.

After the server's verification of the identification information of the to-be-activated health monitoring equipment is passed, the server will return the key set corresponding to the to-be-activated health monitoring equipment to the base station, wherein the key set contains the key and key identification corresponding to the to-be-activated health monitoring equipment.

S310. The designated mobile terminal performs bidirectional authentication with the to-be-activated health monitoring equipment based on the authentication information of the to-be-activated health monitoring equipment. If the authentication succeeds, Step S214 is executed; if the authentication fails, the authentication failure information is returned to the server.

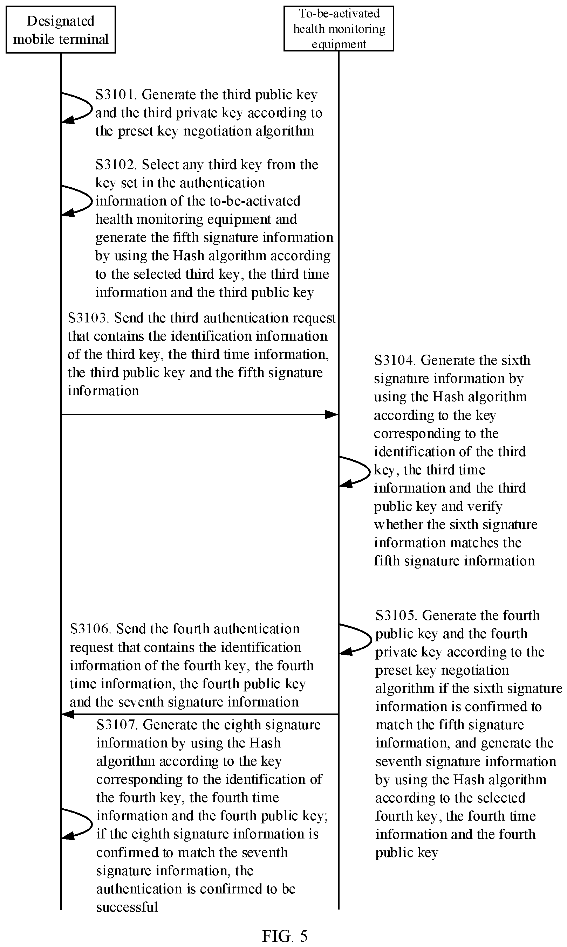

In a specific implementation, the designated mobile terminal and the to-be-activated health monitoring equipment may perform bidirectional authentication according to the process shown in FIG. 5, which may comprise the following steps:
S3101. The designated mobile terminal generates the third public key and the third private key according to the preset key negotiation algorithm.

In a specific implementation, the designated mobile terminal may generate a public key and a corresponding private key according to the ECDH key negotiation algorithm, which may be marked as the third public key and the third private key respectively; the third public key and the third private key may also be generated by any other key negotiation algorithm, which is not defined in the embodiments of the present application. The ECDH key negotiation algorithm is only described as an example in the embodiments of the present application.

S3102. The designated mobile terminal selects any third key from the key set in the authentication information of the to-be-activated health monitoring equipment and generates the fifth signature information by using the Hash algorithm according to the selected third key, the third time information and the third public key.

In a specific implementation, the designated mobile terminal receives the authentication information of the to-be-activated health monitoring equipment returned by the server, selects any key from the key set of the to-be-activated health monitoring equipment contained in the authentication information of the to-be-activated health monitoring equipment, which may be marked as the third key, and conducts signature and generates signature information, which may be marked as the fifth signature information, by using the Hash algorithm according to this third key, the third time information and the third public key, wherein the third time information may be regarded as the current time information.

S3103. The designated mobile terminal sends the to-be-activated health monitoring equipment the third authentication request that contains the identification information of the third key, the third time information, the third public key and the fifth signature information.

S3104. The to-be-activated health monitoring equipment generates the sixth signature information by using the Hash algorithm according to the key corresponding to the identification of the third key, the third time information and the third public key and verifies whether the sixth signature information matches the fifth signature information. If the sixth signature information is confirmed to match the fifth signature information, Step S3105 is executed; otherwise, the verification failure information is returned to the base station.

In a specific implementation, the to-be-activated health monitoring equipment finds the key corresponding to the identification of the third key in the key set stored locally according to the identification of the third key sent by the designated mobile terminal, generates the sixth signature information by using the Hash algorithm according to the key found and the third time information and third public key sent by the designated mobile terminal, and verifies whether the sixth signature information matches the fifth signature information sent by the base station. If the sixth signature information is confirmed to match the fifth signature information, the unidirectional authentication is confirmed to be successful; otherwise, the authentication is confirmed to have failed, the authentication failure information is returned to the designated mobile terminal, and the designated mobile terminal reports the failure information on the authentication with the to-be-activated health monitoring equipment to the server.

S3105. The to-be-activated health monitoring equipment generates the fourth public key and the fourth private key according to the preset key negotiation algorithm and also generates the seventh signature information by using the Hash algorithm according to the selected fourth key, the fourth time information and the fourth public key.

In a specific implementation, the to-be-activated health monitoring equipment generates a public key and a corresponding private key according to the ECDH key negotiation algorithm, which may be marked as the fourth public key and the fourth private key respectively. The to-be-activated health monitoring equipment selects any key from the key set stored locally, which may be marked as the fourth key, and generates the seventh signature information by using the Hash algorithm according to the selected fourth key, the fourth time information and the fourth public key, wherein the fourth time information may be regarded as the current time information.

S3106. The to-be-activated health monitoring equipment sends the designated mobile terminal the fourth authentication request that contains the identification information of the fourth key, the fourth time information, the fourth public key and the seventh signature information.

S3107. The designated mobile terminal generates the eighth signature information by using the Hash algorithm according to the key corresponding to the identification of the fourth key, the fourth time information and the fourth public key; if the eighth signature information is confirmed to match the seventh signature information, the authentication is confirmed to be successful.

In a specific implementation, the designated mobile terminal finds the key corresponding to the identification of the fourth key from the key set of the to-be-activated health monitoring equipment contained in the authentication information of the to-be-activated health monitoring equipment returned by the server, generates the eighth signature information by using the Hash algorithm according to the found key corresponding to the identification of the fourth key and the fourth time information and fourth public key sent by the to-be-activated health monitoring equipment, and verifies whether the eighth signature information matches the seventh signature information sent by the to-be-activated health monitoring equipment. If the eighth signature information is confirmed to match the seventh signature information, the bidirectional authentication between the designated mobile terminal and the to-be-activated health monitoring equipment is confirmed to be successful; otherwise, the authentication is confirmed to have failed, and the failure information on the authentication with the to-be-activated health monitoring equipment is returned to the server.

S311. The designated mobile terminal activates the to-be-activated health monitoring equipment.

In a specific implementation, after the bidirectional authentication between the designated mobile terminal and the to-be-activated health monitoring equipment succeeds, the designated mobile terminal will receive a Bluetooth data packet sent by the to-be-activated health monitoring equipment and establish a Bluetooth connection with the to-be-activated health monitoring equipment to activate the to-be-activated health monitoring equipment.

S312. The activated health monitoring equipment returns the activation success information to the designated mobile terminal.

In this step, the to-be-activated health monitoring equipment returns the activation success information to the designated mobile terminal after being activated.

S313. The designated mobile terminal reports the health monitoring equipment activation success information to the server.

In this step, the designated mobile terminal reports the activation success information of the activated health monitoring equipment to the server.

S314. The server updates the status of the health monitoring equipment as "activated".

In this step, the server updates the status of the activated health monitoring equipment as "updated" and saves the status.

S315. The server returns the uploaded result notification to the designated mobile terminal.

S316. The designated mobile terminal breaks the Bluetooth connection with the activated health monitoring equipment.

Then, the health monitoring equipment returns the disconnection success notification to the designated mobile terminal, marking the completion of the activation process of the health monitoring equipment.

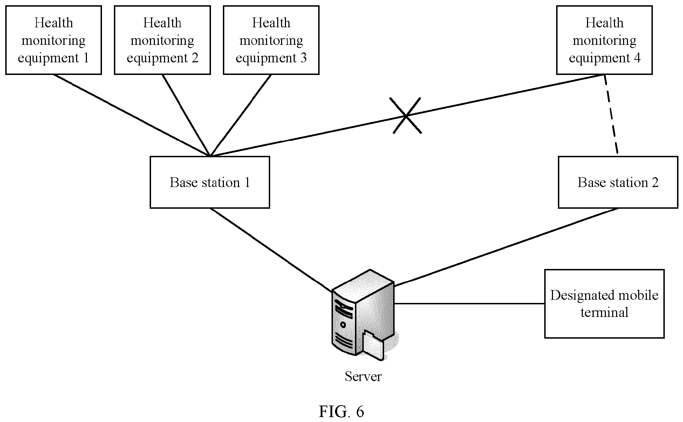

There are the following two application scenarios for the disconnection status of the health monitoring equipment:
One application scenario for the disconnection status of the health monitoring equipment is as follows: After being activated, the health monitoring equipment is not connected to any base station to upload the health monitoring data on the monitored object and thus is in a disconnection status. The other application scenario is as follows: The health monitoring equipment has been connected to a base station and uploads the health monitoring data on the monitored object to the base station but is disconnected from the base station due to the shift of the location of the monitored object wearing the health monitoring equipment, thus the health monitoring equipment is in a disconnection status. In the application scenario shown in FIG. 6, health monitoring equipment 1, 2, 3 and 4 are connected to base station 1 and upload their health monitoring data to the server via base station 1, but the monitored object wearing health monitoring equipment 4 shifts from the area covered by base station 1 to that covered by base station 2, consequently, heath monitoring equipment 4 is disconnected from base station 1.

Based on the aforementioned two disconnection statuses of the health monitoring equipment, the health monitoring equipment may continuously send out a Bluetooth data packet to connect to the best base station that is connectable nearby and then upload the health monitoring data on the monitored object to the base station.

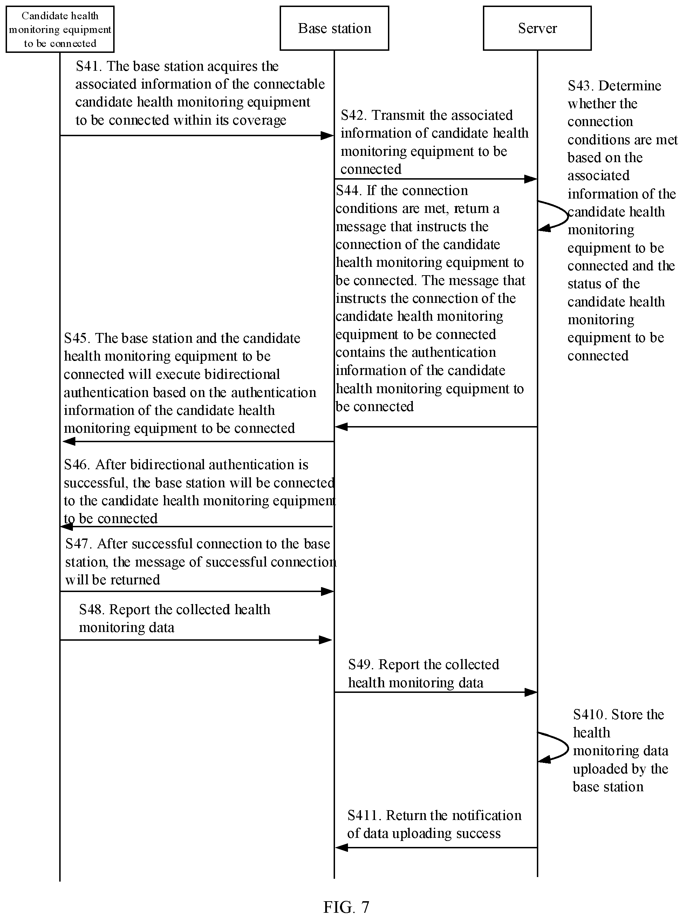

FIG. 7 illustrates a schematic diagram for the implementation process of the data transmission method as provided in the embodiment of the present application, which may comprise the following steps:
S41. The base station acquires the associated information of the connectable candidate health monitoring equipment to be connected within its coverage.

In a specific implementation, the base station receives the associated information of candidate health monitoring equipment to be connected that is sent via Bluetooth by the connectable candidate health monitoring equipment to be connected within its coverage, wherein the associated information of the candidate health monitoring equipment to be connected may include but is not limited to the following: the identification information of the candidate health monitoring equipment to be connected, and the signal strength information between the candidate health monitoring equipment to be connected and the base station, wherein, the signal strength information between the candidate health monitoring equipment to be connected and the base station may be, but is not limited to, a Received Signal Strength Indication (RSSI).

In one embodiment, the associated information of the candidate health monitoring equipment to be connected may also include its service quality capability information, wherein the service quality capability information may include such information as the manufacturer information and model information of the candidate health monitoring equipment to be connected, which is not defined in the embodiment of the present application.

S42. The base station sends the associated information of candidate health monitoring equipment to be connected to the server.

In an implementation, if the base station performs data interaction with the server for the first time, the base station first logs in to the server and then sends the associated information of candidate health monitoring equipment to be connected to the server.

Refer to Steps S21 ~ S23 for the specific process for the base station to log in to the server, and the description thereof is omitted here.

S43. The server determines whether the connection conditions are met based on the associated information of the candidate health monitoring equipment to be connected and the status of the candidate health monitoring equipment to be connected. If the connection conditions are met, Step S44 is executed; otherwise, the notification message that the candidate health monitoring equipment to be connected does not meet the connection conditions is returned to the base station.

In a specific implementation, in one embodiment, the following method may be adopted to confirm that the candidate health monitoring equipment to be connected meets the connection conditions:
The server searches for the status of the candidate health monitoring equipment to be connected based on the identification of the equipment. If the candidate health monitoring equipment to be connected is confirmed to be in an activated status and the signal strength between the equipment and the base station at the present moment is greater than that between the equipment and the first base station at the moment the equipment was disconnected from the first base station to which the equipment was connected last time, the equipment is confirmed to meet the connection conditions.

Specifically, when any health monitoring equipment that has been successfully connected to any base station to upload health monitoring data is disconnected from the base station, the base station will upload the signal strength (i.e. RSSI) between the health monitoring equipment and the base station at the disconnection moment to the server, and the server will store the signal strength and disconnection time information between the health monitoring equipment and the base station.

In an implementation, when the server receives the associated information of the candidate health monitoring equipment to be connected sent by the base station, the server may check whether the candidate health monitoring equipment to be connected has been activated or not according to the identification of the equipment and compare the RSSI between the equipment and the base station at the present moment with the RSSI between the equipment and the first base station at the moment the equipment was disconnected from the first base station to which the equipment was connected last time. If the candidate health monitoring equipment to be connected is confirmed to have been activated and the signal strength between the equipment and the base station at the present moment is greater than that between the equipment and the first base station at the moment the equipment was disconnected from the first base station to which the equipment was connected last time, the equipment is confirmed to meet the connection conditions.

Alternatively, the following method may also be used to confirm that the connection conditions are met:
The server searches for the status of the candidate health monitoring equipment to be connected based on the identification of the equipment. If the candidate health monitoring equipment to be connected is confirmed to be in an activated status, and the signal strength between the equipment and the base station at the present moment is greater than that between the equipment and each second base station adjacent to the base station, the equipment is confirmed to meet the connection conditions.

Specifically, the server may check whether the candidate health monitoring equipment to be connected has been activated or not according to the identification of the equipment and compare the RSSI between the equipment and the base station at the present moment with the signal strength between the equipment and each second base station adjacent to the base station. If the candidate health monitoring equipment to be connected is confirmed to have been activated, and the signal strength between the equipment and the base station at the present moment is greater than that between the equipment and each second base station adjacent to the base station, the equipment is confirmed to meet the connection conditions.

In one embodiment, the server may also store information that can measure the service quality capability of the health monitoring equipment, such as the set manufacturer information, the set model information of the health monitoring equipment and other information. The embodiment of the present application may also adopt the following method to confirm that the connection conditions are met:
The server searches for the status of the candidate health monitoring equipment to be connected based on the identification of the equipment. If the candidate health monitoring equipment to be connected is confirmed to be in an activated status, the manufacturer of the equipment is the set manufacturer (or the model of the equipment is the set model), and the signal strength between the equipment and the base station at the present moment is greater than that between the equipment and the first base station at the moment the equipment was disconnected from the first base station to which the equipment was connected last time, the equipment is confirmed to meet the connection conditions.

Alternatively, the following method may also be used to confirm that the connection conditions are met:
The server searches for the status of the candidate health monitoring equipment to be connected based on the identification of the equipment. If the candidate health monitoring equipment to be connected is confirmed to be in an activated status, the manufacturer of the equipment is the set manufacturer (or the model of the equipment is the set model), and the signal strength between the equipment and the base station at the present moment is greater than that between the equipment and each second base station adjacent to the base station, the equipment is confirmed to meet the connection conditions.

S44. The server returns the message that instructs connection of the candidate health monitoring equipment to be connected to the base station. The message that instructs connection of the candidate health monitoring equipment to be connected contains the authentication information of the candidate health monitoring equipment to be connected.

During the transmission of the health monitoring data of the monitored object collected by the health monitoring equipment, if the health monitoring data is leaked or tampered with, serious impacts may be caused to the life, property, health, etc. of the monitored object. To improve the security of data transmission, before the establishment of a connection between the base station and the candidate health monitoring equipment to be connected that meets the connection conditions, the base station and the candidate health monitoring equipment to be connected may perform bidirectional authentication first and generate a shared key for encrypted transmission during the subsequent transmission of the health monitoring data.

In a specific implementation, the authentication information of the candidate health monitoring equipment to be connected includes the key set corresponding to the candidate health monitoring equipment to be connected, and the key set of the candidate health monitoring equipment to be connected includes the keys and key identification corresponding to the candidate health monitoring equipment to be connected.

S45. The base station and candidate health monitoring equipment to be connected execute bidirectional authentication based on the authentication information of the candidate health monitoring equipment to be connected.

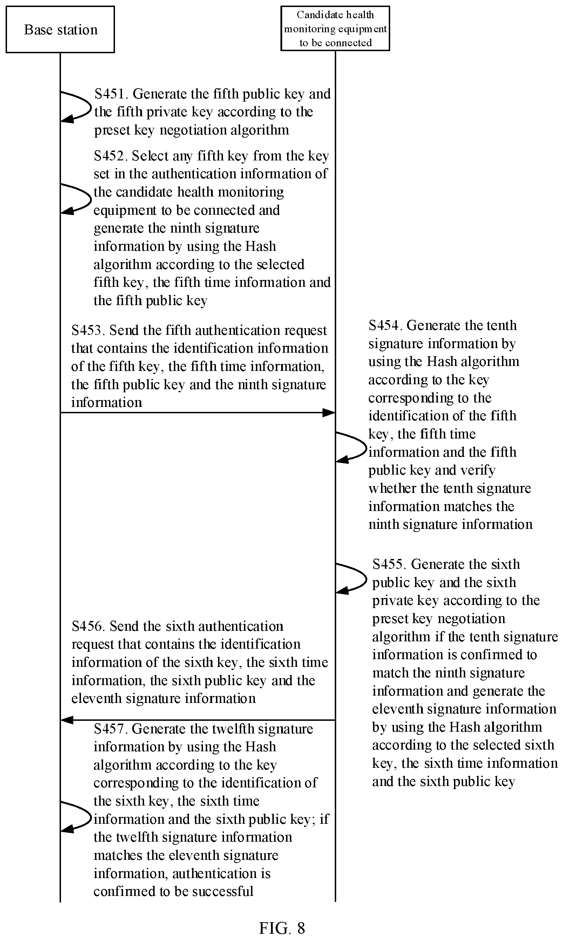

In a specific implementation, the base station and candidate health monitoring equipment to be connected may perform bidirectional authentication according to the process shown in FIG. 8, which may comprise the following steps:
5451. The base station generates the fifth public key and the fifth private key according to the preset key negotiation algorithm.

In a specific implementation, the base station may generate a public key and a corresponding private key according to the ECDH key negotiation algorithm, which may be marked as the fifth public key and the fifth private key respectively; the fifth public key and the fifth private key may also be generated by any other key negotiation algorithm, which is not defined in the embodiments of the present application. The ECDH key negotiation algorithm is only described as an example in the embodiments of the present application.

S452. The base station selects any fifth key from the key set in the authentication information of the candidate health monitoring equipment to be connected and generates the ninth signature information by using the Hash algorithm according to the selected fifth key, the fifth time information and the fifth public key.

In a specific implementation, the base station receives the authentication information of the candidate health monitoring equipment to be connected that is returned by the server, selects any key from the key set of the candidate health monitoring equipment to be connected that is contained in the authentication information of the candidate health monitoring equipment to be connected, which may be marked as the fifth key, and conducts signature and generates signature information, which may be marked as the ninth signature information, by using the Hash algorithm according to the fifth key, the fifth time information and the fifth public key, wherein the fifth time information may be regarded as the current time information.

S453. The base station sends the candidate health monitoring equipment to be connected the fifth authentication request that contains the identification information of the fifth key, the fifth time information, the fifth public key and the ninth signature information.

S454. The candidate health monitoring equipment to be connected generates the tenth signature information by using the Hash algorithm according to the key corresponding to the identification of the fifth key, the fifth time information and the fifth public key and verifies whether the tenth signature information matches the ninth signature information. If the tenth signature information is confirmed to match the ninth signature information, Step S455 is executed; otherwise, the verification failure information is returned to the base station.

In a specific implementation, the candidate health monitoring equipment to be connected finds the key corresponding to the identification of the fifth key in the key set stored locally according to the identification of the fifth key sent by the base station, generates the tenth signature information by using the Hash algorithm according to the key found and the fifth time information and fifth public key sent by the base station, and verifies whether the tenth signature information matches the ninth signature information sent by the base station. If the tenth signature information is confirmed to match the ninth signature information, the unidirectional authentication is confirmed to be successful; otherwise, the authentication is confirmed to have failed, the authentication failure information is returned to the base station, and the base station reports the failure information on the authentication with the candidate health monitoring equipment to be connected to the server.

S455. The candidate health monitoring equipment to be connected generates the sixth public key and the sixth private key according to the preset key negotiation algorithm and also generates the eleventh signature information by using the Hash algorithm according to the selected sixth key, the sixth time information and the sixth public key.

In a specific implementation, the candidate health monitoring equipment to be connected generates a public key and a corresponding private key according to the ECDH key negotiation algorithm, which may be marked as the sixth public key and the sixth private key respectively. The candidate health monitoring equipment to be connected selects any key from the key set stored locally, which may be marked as the sixth key, and generates the eleventh signature information by using the Hash algorithm according to the selected sixth key, the sixth time information and the sixth public key, wherein the sixth time information may be regarded as the current time information.

S456. The candidate health monitoring equipment to be connected sends the base station the sixth authentication request that contains the identification information of the sixth key, the sixth time information, the sixth public key and the eleventh signature information.

S457. The base station generates the twelfth signature information by using the Hash algorithm according to the key corresponding to the identification of the sixth key, the sixth time information and the sixth public key; if the twelfth signature information matches the eleventh signature information, authentication is confirmed to be successful.

In a specific implementation, the base station finds the key corresponding to the identification of the sixth key from the key set of the candidate health monitoring equipment to be connected contained in the authentication information of the candidate health monitoring equipment to be connected returned by the server, generates the twelfth signature information by using the Hash algorithm according to the found key corresponding to the identification of the sixth key and the fourth time information and fourth public key sent from the candidate health monitoring equipment to be connected, and verifies whether the twelfth signature information matches the eleventh signature information sent from the candidate health monitoring equipment to be connected. If the twelfth signature information is confirmed to match the eleventh signature information, the bidirectional authentication between the base station and the candidate health monitoring equipment to be connected is confirmed to be successful; otherwise, the authentication is confirmed to have failed, and the base station will return the failure information on the authentication with the candidate health monitoring equipment to be connected to the server.

Further, if the bidirectional authentication is confirmed to be successful, the following may also be included:
The candidate health monitoring equipment to be connected and the base station generate a shared key by the preset key negotiation algorithm based on the fifth public key, the fifth private key corresponding to the fifth public key, the sixth public key and the sixth private key and send the shared key to the server. Then, the server stores the correspondence between the identification information of the health monitoring equipment and the shared key.

In a specific implementation, when the bidirectional authentication between the base station and the candidate health monitoring equipment to be connected succeeds, the base station and the candidate health monitoring equipment to be connected also generate a shared key by using the ECDH key negotiation algorithm based on the fifth public key, the fifth private key corresponding to the fifth public key, the sixth public key and the sixth private key.

Specifically, the base station generates a key according to the fifth public key, the sixth private key and the ECDH key negotiation algorithm. The candidate health monitoring equipment to be connected generates a key according to the sixth public key, the fifth private key and the ECDH key negotiation algorithm. The two keys are the same, namely shared keys, and then the base station sends the shared key to the server. After receiving the shared key sent from the base station, the server stores the correspondence between the identification information of the candidate health monitoring equipment to be connected (also, i.e. the MAC address information of the to-be-activated health monitoring equipment) and the shared key.

S46. After bidirectional authentication is successful, the base station will be connected to the candidate health monitoring equipment to be connected.

In this step, a Bluetooth connection is established after the bidirectional authentication between the base station and the candidate health monitoring equipment to be connected succeeds.

S47. After the successful connection of the health monitoring equipment to the base station, the message of successful connection will be returned to the base station.

S48. The health monitoring equipment reports the collected health monitoring data to the base station.

In a specific implementation, the health monitoring equipment encrypts the health monitoring data collected in real time according to the shared key negotiated with the base station and the preset encryption algorithm to generate encrypted health monitoring data and sends the encrypted health monitoring data to the base station, wherein the preset encryption algorithm may be but is not limited to the symmetric encryption algorithm, such as the Advanced Encryption Standard (AES) encryption algorithm, which is not defined in the embodiment of the present application.

S49. The base station sends the health monitoring data uploaded by the health monitoring equipment to the server.

In a specific implementation, the base station receives the encrypted health monitoring data uploaded by the health monitoring equipment and then uploads the same to the server.

Upon receiving the encrypted health monitoring data uploaded by the health monitoring equipment, the base station may also adopt the shared key negotiated with the health monitoring equipment for decryption based on the preset encryption algorithm, process the decrypted health monitoring data to a certain extent, such as processing the format of the health monitoring data into the data format supported by the server, and then encrypt and upload to the server the processed health monitoring data according to the shared key and the preset encryption algorithm.

S410. The server stores the health monitoring data uploaded by the base station.

In a specific implementation, the encrypted health monitoring data uploaded by the base station to the server also includes the identification information of the health monitoring equipment. The server finds the corresponding shared key according to the identification of the health monitoring equipment. The server decrypts the encrypted health monitoring data according to the shared key and stores the decrypted health monitoring data.

S411. The server returns the notification of data uploading success to the base station.

Thus, after the health monitoring data is uploaded to the server, a medical worker may log in to the server via the designated mobile terminal to view and download the health monitoring data of the monitored object in real time; consequently, the health monitoring data is viewed and monitored in real time.

The data transmission method provided in the embodiment of the present application activates the health monitoring equipment via the designated mobile terminal or base station. After the health monitoring equipment is activated successfully and the health monitoring equipment activation success information is sent to the server, the server marks the status of the health monitoring equipment as "activated". The base station detects the connectable health monitoring equipment to be connected within its coverage, designates the health monitoring equipment to be connected as candidate health monitoring equipment to be connected, and sends the associated information of the candidate health monitoring equipment to be connected to the server. If the server determines the connection conditions are met based on the associated information of the candidate health monitoring equipment to be connected and the status of the candidate health monitoring equipment to be connected, it will return the message that instructs connection of the candidate health monitoring equipment to be connected to the base station. The base station will be connected to the candidate health monitoring equipment to be connected and will send the health monitoring data collected from the connected health monitoring equipment to the server. In the embodiment of the present application, the mobile terminal of the monitored object is not needed to activate health monitoring equipment or upload the health monitoring data monitored by the health monitoring equipment to the server. Instead, a designated mobile terminal (e.g. the mobile terminal of a medical worker or a separate mobile terminal) or the base station is used to activate the health monitoring equipment, and the base station will detect the connectable candidate health monitoring equipment to be connected within its coverage. The server will determine whether the candidate health monitoring equipment to be connected meets the connection conditions. The base station will be connected to the candidate health monitoring equipment to be connected that meets the connection conditions. The base station can in real time upload the health monitoring data collected from the health monitoring equipment to the server. This will solve the problem where the health monitoring equipment cannot be activated and the health monitoring data cannot be uploaded because the monitored object does not have a mobile phone or any other mobile terminal or does not know how to use the health monitoring application program in the mobile terminal. Besides, the base station provided in the embodiment of the present application will in real time detect the connectable health monitoring equipment within its coverage. If the monitored object wearing the health monitoring equipment moves and such movement results in communication disconnection between the health monitoring equipment and the connected base station, the equipment will be immediately detected by the base station whose coverage reaches the health monitoring equipment, so the equipment will be connected to the base station to continue uploading the health monitoring data in real time.

Based on the same inventive concept, the embodiment of the present application further provides a data transmission method implemented at the server. As the principle of the data transmission method implemented at the server to solve a problem is similar to that of the aforementioned data transmission method, the data transmission method implemented at the server may be implemented by reference to the implementation of the aforementioned data transmission method, which is not described to avoid repetition.

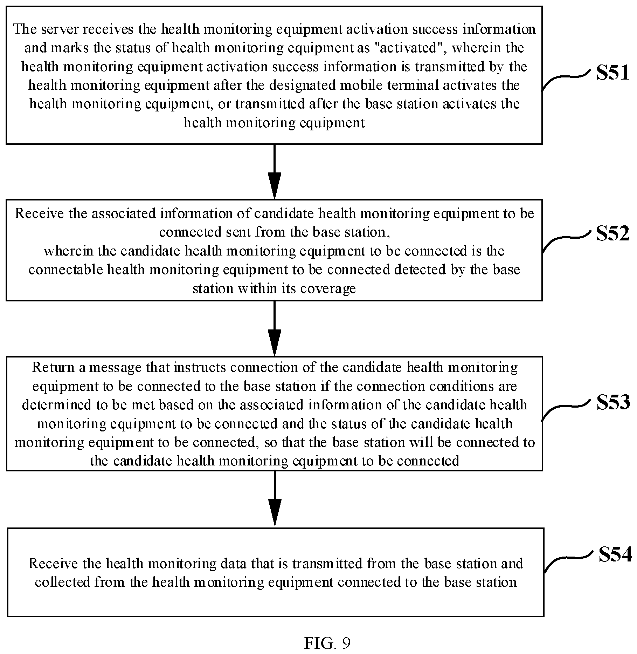

FIG. 9 illustrates a schematic diagram for the implementation process of the data transmission method implemented at the server as provided in the embodiment of the present application, which may comprise the following steps:
S51. The server receives the health monitoring equipment activation success information and marks the status of health monitoring equipment as "activated", wherein the health monitoring equipment activation success information is transmitted by the health monitoring equipment after the designated mobile terminal activates the health monitoring equipment, or transmitted after the base station activates the health monitoring equipment.

S52. Receive the associated information of the candidate health monitoring equipment to be connected sent from the base station, wherein the candidate health monitoring equipment to be connected is the connectable health monitoring equipment to be connected detected by the base station within its coverage.

S53. Return a message that instructs connection of the candidate health monitoring equipment to be connected to the base station if the connection conditions are determined to be met based on the associated information of the candidate health monitoring equipment to be connected and the status of the candidate health monitoring equipment to be connected, so that the base station will be connected to the candidate health monitoring equipment to be connected.

S54. Receive the health monitoring data that is transmitted from the base station and collected from the health monitoring equipment connected to the base station.

In one embodiment, the associated information of the candidate health monitoring equipment to be connected includes the identification information of the candidate health monitoring equipment to be connected and the signal strength between the candidate health monitoring equipment to be connected and the base station;
determining whether the connection conditions are met based on the associated information of the candidate health monitoring equipment to be connected and the status of the candidate health monitoring equipment to be connected, specifically comprising:
searching for the status of the candidate health monitoring equipment to be connected based on the identification of the candidate health monitoring equipment to be connected;
confirming the connection conditions are met if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the first base station at the moment the equipment was disconnected from the base station to which it was connected last time.

In one embodiment, the associated information of the candidate health monitoring equipment to be connected includes the identification information of the candidate health monitoring equipment to be connected and the signal strength between the candidate health monitoring equipment to be connected and the base station;
determining whether the connection conditions are met based on the associated information of the candidate health monitoring equipment to be connected and the status of the candidate health monitoring equipment to be connected, specifically comprising:
searching for the status of the candidate health monitoring equipment to be connected based on the identification of the candidate health monitoring equipment to be connected;
confirming the connection conditions are met if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the second base station adjacent to the base station.

In one embodiment, the message that instructs the connection of the candidate health monitoring equipment contains the authentication information of the candidate health monitoring equipment to be connected, so that the base station and the candidate health monitoring equipment to be connected will execute bidirectional authentication based on the authentication information, and they will connect after successful authentication.

In one embodiment, the authentication information of the candidate health monitoring equipment to be connected contains the key set corresponding to the candidate health monitoring equipment to be connected, and the key set contains the keys and key identification corresponding to the candidate health monitoring equipment to be connected.

In one embodiment, before the associated information of the candidate health monitoring equipment to be connected transmitted from the base station is received, further comprising:
receiving the login request sent from the base station, wherein the login request contains the identification information and login key information of the base station;
verifying the identification information and login key information of the base station; returning the login token information to the base station if verification is passed, so that the base station will carry the login token when sending the data.

Based on the same inventive concept, the embodiment of the present application further provides a data transmission device implemented at the server. As the principle of the data transmission device implemented at the server to solve a problem is similar to that of the aforementioned data transmission method, the data transmission device implemented at the server may be implemented by reference to the implementation of the aforementioned data transmission method, which is not described to avoid repetition.

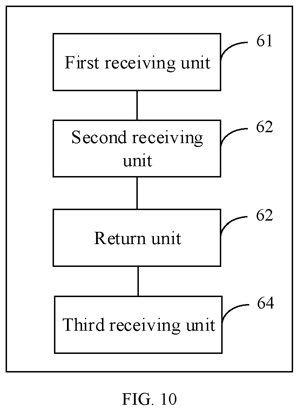

FIG. 10 illustrates a structural schematic diagram for the data transmission device implemented at the server as provided in the embodiment of the present application, which may comprise:
a first receiving unit 61, used to receive the health monitoring equipment activation success information and mark the status of the health monitoring equipment as "activated", wherein the health monitoring equipment activation success information is sent from the health monitoring equipment after the designated mobile terminal activates the health monitoring equipment or is sent after the base station activates the health monitoring equipment;
a second receiving unit 62, used to receive the associated information of candidate health monitoring equipment to be connected, wherein the candidate health monitoring equipment to be connected is the connectable health monitoring equipment to be connected detected by the base station within its coverage;
a return unit 63, used to return a message that instructs connection of the candidate health monitoring equipment to be connected to the base station if the connection conditions are determined to be met based on the associated information of the candidate health monitoring equipment to be connected and the status of the candidate health monitoring equipment to be connected, so that the base station will be connected to the candidate health monitoring equipment to be connected; and
a third receiving unit 64, used to receive the health monitoring data that is transmitted from the base station and collected from the health monitoring equipment connected to the base station.

In one embodiment, the associated information of the candidate health monitoring equipment to be connected includes the identification information of the candidate health monitoring equipment to be connected and the signal strength between the candidate health monitoring equipment to be connected and the base station;

The return unit 63 is specifically used to search for the status of the candidate health monitoring equipment to be connected based on the identification of the candidate health monitoring equipment to be connected; if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the first base station at the moment the equipment was disconnected from the first base station to which it was connected last time, the connection conditions are confirmed to be met.

In one embodiment, the associated information of the candidate health monitoring equipment to be connected includes the identification information of the candidate health monitoring equipment to be connected and the signal strength between the candidate health monitoring equipment to be connected and the base station;

The return unit 63 is specifically used to search for the status of the candidate health monitoring equipment to be connected based on the identification of the candidate health monitoring equipment to be connected; if the status of the candidate health monitoring equipment to be connected is confirmed to be activated, and the signal strength between the candidate health monitoring equipment to be connected and the base station at the present moment is greater than the signal strength between the candidate health monitoring equipment to be connected and each second base station adjacent to the base station, the connection conditions are confirmed to be met.

In one embodiment, the message that instructs the connection of the candidate health monitoring equipment contains the authentication information of the candidate health monitoring equipment to be connected, so that the base station and the candidate health monitoring equipment to be connected will execute bidirectional authentication based on the authentication information, and they will connect after successful authentication.

In one embodiment, the authentication information of the candidate health monitoring equipment to be connected contains the key set corresponding to the candidate health monitoring equipment to be connected, and the key set contains the keys and key identification corresponding to the candidate health monitoring equipment to be connected.

In one embodiment, the device further comprises:
a fourth receiving unit, used to receive the login request sent from the base station before the associated information of candidate health monitoring equipment to be connected is received from the base station, wherein the login request contains the identification information and login key information of the base station;
a verification unit, used to verify the identification information and login key information of the base station, and if verification is passed, return the login token information to the base station, so that the base station will carry the login token when sending the data.

Based on the same inventive concept, the embodiment of the present application further provides a data transmission method implemented at the base station. As the principle of the data transmission method implemented at the base station to solve a problem is similar to that of the aforementioned data transmission method, the data transmission method implemented at the base station may be implemented by reference to the implementation of the aforementioned data transmission method, which is not described to avoid repetition.

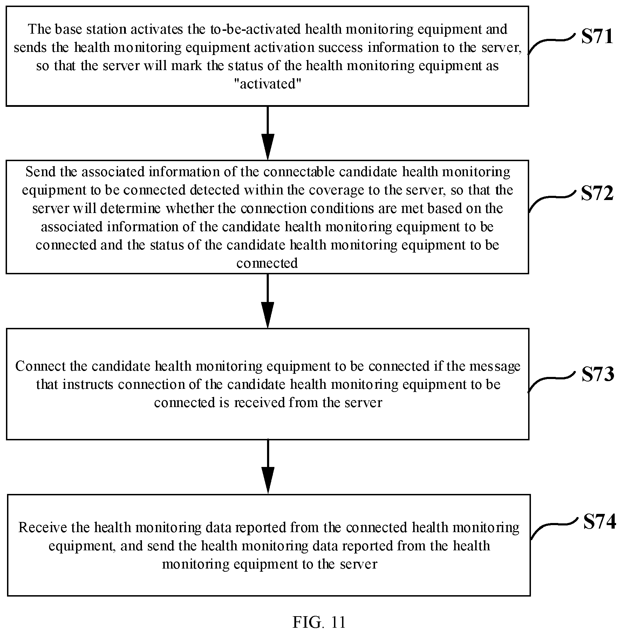

FIG. 11 illustrates a schematic diagram for the implementation process of the data transmission method implemented at the base station as provided in the embodiment of the present application, which may comprise the following steps:
S71. The base station activates the to-be-activated health monitoring equipment and sends the health monitoring equipment activation success information to the server, so that the server will mark the status of the health monitoring equipment as "activated".

S72. Send the associated information of the connectable candidate health monitoring equipment to be connected detected within the coverage to the server, so that the server will determine whether the connection conditions are met based on the associated information of the candidate health monitoring equipment to be connected and the status of the candidate health monitoring equipment to be connected.

S73. Connect the candidate health monitoring equipment to be connected if the message that instructs connection of the candidate health monitoring equipment to be connected is received from the server.

S74. Receive the health monitoring data reported from the connected health monitoring equipment, and send the health monitoring data reported from the health monitoring equipment to the server.

In one embodiment, the to-be-activated health monitoring equipment will be activated in the following ways:
receiving the Bluetooth broadcast data packet sent from the to-be-activated health monitoring equipment, wherein the Bluetooth broadcast data packet is sent after the to-be-activated health monitoring equipment is woken up by the designated mobile terminal;
obtaining the identification information of the to-be-activated health monitoring equipment from the to-be-activated health monitoring equipment;
sending the identification information of the to-be-activated health monitoring equipment to the server, so that the server will verify the identification information of the to-be-activated health monitoring equipment;
receiving the authentication information of the to-be-activated health monitoring equipment sent from the server, wherein the authentication information of the to-be-activated health monitoring equipment is sent after the server verifies the identification information of the to-be-activated health monitoring equipment;
executing bidirectional authentication with the to-be-activated health monitoring equipment based on the authentication information of the to-be-activated health monitoring equipment, and activating the to-be-activated health monitoring equipment after authentication is successful.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
executing bidirectional authentication with the to-be-activated health monitoring equipment based on the authentication information of the to-be-activated health monitoring equipment, specifically comprising:
generating the first public key and first private key according to the preset key negotiation algorithm;
selecting any first key from the key set, and generating the first signature information by using the Hash algorithm according to the selected first key, first time information and the first public key;
sending the first authentication request to the to-be-activated health monitoring equipment, wherein the first authentication request contains the identification information of the first key, the first time information, the first public key and the first signature information, so that the to-be-activated health monitoring equipment will generate the second signature information by using the Hash algorithm according to the key corresponding to the identification of the first key, the first time information and the first public key, so as to verify whether the second signature information matches the first signature information;
receiving the second authentication request sent when the to-be-activated health monitoring equipment confirms the second signature information matches the first signature information, wherein the second authentication request contains the identification information of the second key, second time information, second public key and third signature information, the third signature information is generated by the to-be-activated health monitoring equipment by using the Hash algorithm according to the selected second key, the second time information and the second public key and the second public key is generated by the to-be-activated health monitoring equipment by using the preset key negotiation algorithm;
generating the fourth signature information by using the Hash algorithm according to the key corresponding to the identification of the second key, the second time information and the second public key;
confirming the authentication is successful if the fourth signature information is confirmed to match the third signature information.

Based on the same inventive concept, the embodiment of the present application further provides a data transmission device implemented at the base station. As the principle of the data transmission device implemented at the base station to solve a problem is similar to that of the aforementioned data transmission method, the data transmission device implemented at the base station may be implemented by reference to the implementation of the aforementioned data transmission method, which is not described to avoid repetition.

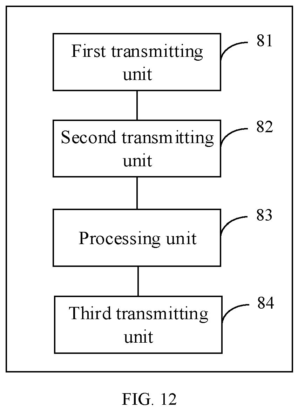

FIG. 12 illustrates a structural schematic diagram for the data transmission device implemented at the base station as provided in the embodiment of the present application, which may comprise:
a first transmitting unit 81, used to send the health monitoring equipment activation success information to the server after the to-be-activated health monitoring equipment is activated, so that the server will mark the status of the health monitoring equipment as "activated";
a second transmitting unit 82, used to send the associated information of the connectable candidate health monitoring equipment to be connected detected within the coverage to the server, so that the server will determine whether the candidate health monitoring equipment to be connected meets the connection conditions or not based on the associated information and status of the candidate health monitoring equipment to be connected;
a processing unit 83, used to connect the candidate health monitoring equipment to be connected if the message that instructs connection of the candidate health monitoring equipment to be connected is received from the server; and
a third transmitting unit 84, used to receive the health monitoring data reported from the connected health monitoring equipment and send the health monitoring data reported from the health monitoring equipment to the server.

In one embodiment, the device further comprises:
an activation unit, used to activate the to-be-activated health monitoring equipment by means of: receiving the Bluetooth broadcast data packet sent from the to-be-activated health monitoring equipment, wherein the Bluetooth broadcast data packet being sent after the to-be-activated health monitoring equipment is woken up by the designated mobile terminal; obtaining the identification information of the to-be-activated health monitoring equipment from the to-be-activated health monitoring equipment; sending the identification information of the to-be-activated health monitoring equipment to the server, so that the server will verify the identification information of the to-be-activated health monitoring equipment; receiving the authentication information of the to-be-activated health monitoring equipment sent from the server, wherein the authentication information of the to-be-activated health monitoring equipment sent from the server is sent after the server verifies the identification information of the to-be-activated health monitoring equipment; executing bidirectional authentication with the to-be-activated health monitoring equipment based on the authentication information of the to-be-activated health monitoring equipment, and activating the to-be-activated health monitoring equipment after authentication is successful.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
the activation unit is specifically used to generate the first public key and the first private key according to the preset key negotiation algorithm; select any first private key from the key set, and generate the first signature information by using the Hash algorithm according to the selected first private key, first time information and the first public key; send the first authentication request to the to-be-activated health monitoring equipment, wherein the first authentication request contains the identification information of the first key, the first time information, the first public key and the first signature information, so that the to-be-activated health monitoring equipment will generate the second signature information by using the Hash algorithm according to the key corresponding to the identification of the first key, the first time information and the first public key, so as to verify whether the second signature information matches the first signature information; receive the second authentication request that is sent when the to-be-activated health monitoring equipment confirms the second signature information matches the first signature information, wherein the second authentication request contains the identification information of the second key, second time information, second public key and third signature information, the third signature information is generated by the to-be-activated health monitoring equipment by using the Hash algorithm according to the selected second key, the second time information and the second public key, and the second public key is generated by the to-be-activated health monitoring equipment by using the preset key negotiation algorithm; generate the fourth signature information by using the Hash algorithm according to the key corresponding to the identification of the second key, the second time information and the second public key; confirm authentication is successful if the fourth signature information is confirmed to match the third signature information.

Based on the same inventive concept, the embodiment of the present application further provides a data transmission method implemented at the designated mobile terminal. As the principle of the data transmission method implemented at the designated mobile terminal to solve a problem is similar to that of the aforementioned data transmission method, the data transmission method implemented at the designated mobile terminal may be implemented by reference to the implementation of the aforementioned data transmission method, which is not described to avoid repetition.

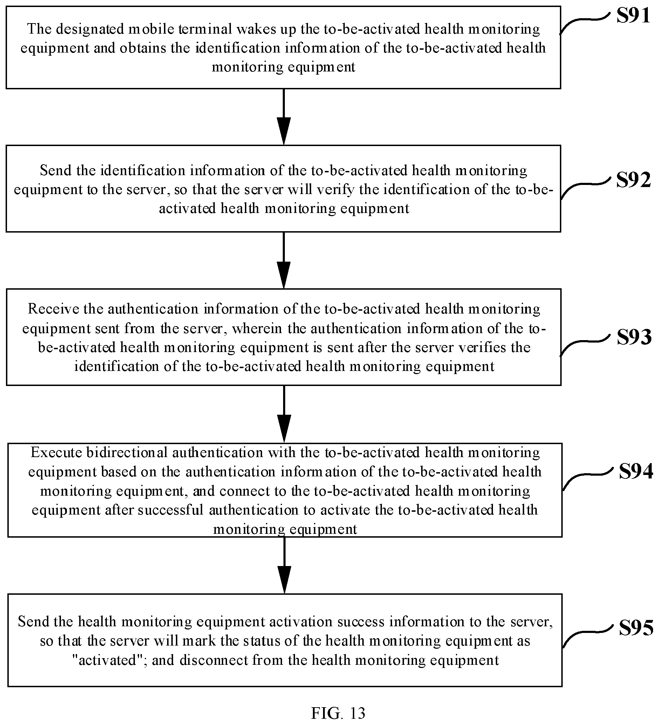

FIG. 13 illustrates a schematic diagram for the implementation process of the data transmission method implemented at the designated mobile terminal as provided in the embodiment of the present application, which may comprise the following steps:
S91. The designated mobile terminal wakes up the to-be-activated health monitoring equipment and obtains the identification information of the to-be-activated health monitoring equipment.

S92. Send the identification information of the to-be-activated health monitoring equipment to the server, so that the server will verify the identification of the to-be-activated health monitoring equipment.

S93. Receive the authentication information of the to-be-activated health monitoring equipment sent from the server, wherein the authentication information of the to-be-activated health monitoring equipment is sent after the server verifies the identification of the to-be-activated health monitoring equipment.

S94. Execute bidirectional authentication with the to-be-activated health monitoring equipment based on the authentication information of the to-be-activated health monitoring equipment, and connect to the to-be-activated health monitoring equipment after successful authentication to activate the to-be-activated health monitoring equipment.

S95. Send the health monitoring equipment activation success information to the server, so that the server will mark the status of the health monitoring equipment as "activated"; and disconnect from the health monitoring equipment.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;
executing bidirectional authentication with the to-be-activated health monitoring equipment based on the authentication information of the to-be-activated health monitoring equipment, specifically comprising:
generating the third public key and third private key according to the preset key negotiation algorithm;
selecting any third key from the key set, and generating the fifth signature information by using the Hash algorithm according to the selected third key, third time information and the third public key;
sending the third authentication request to the to-be-activated health monitoring equipment, wherein the third authentication request contains the identification information of the third key, the third time information, the third public key and the fifth signature information, so that the to-be-activated health monitoring equipment will generate the sixth signature information by using the Hash algorithm according to the key corresponding to the identification of the third key, the third time information and the third public key, so as to verify whether the sixth signature information matches the fifth signature information;
receiving the fourth authentication request sent when the to-be-activated health monitoring equipment confirms the sixth signature information matches the fifth signature information, wherein the fourth authentication request contains the identification information of the fourth key, fourth time information, fourth public key and seventh signature information, the seventh signature information is generated by the to-be-activated health monitoring equipment by using the Hash algorithm according to the selected fourth key, the fourth time information and the fourth public key, and the fourth public key is generated by the to-be-activated health monitoring equipment according to the preset key negotiation algorithm;
generating the eighth signature information by using the Hash algorithm according to the key corresponding to the identification of the fourth key, the fourth time information and the fourth public key;
confirming the authentication is successful if the eighth signature information is confirmed to match the seventh signature information.

In one embodiment, before the identification information of the to-be-activated health monitoring equipment is sent to the server, further comprising:
sending a login request to the server, wherein the login request contains the login information;
receiving the access token information returned from the server, wherein the access token is generated by the server after the login information is successfully verified.

Based on the same inventive concept, the embodiment of the present application further provides a data transmission device implemented at the designated mobile terminal. As the principle of the data transmission device implemented at the designated mobile terminal to solve a problem is similar to that of the aforementioned data transmission method, the data transmission device implemented at the designated mobile terminal may be implemented by reference to the implementation of the aforementioned data transmission method, which is not described to avoid repetition.

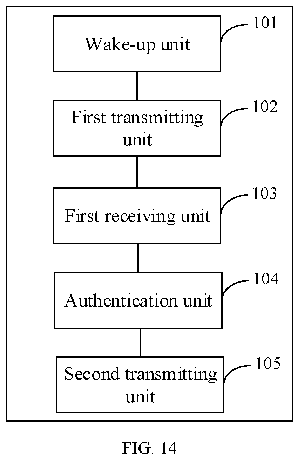

FIG. 14 illustrates a structural schematic diagram for the data transmission device implemented at the designated mobile terminal as provided in the embodiment of the present application, which may comprise:
a wake-up unit 101, used to wake up the to-be-activated health monitoring equipment and obtain the identification information of the to-be-activated health monitoring equipment;
a first transmitting unit 102, used to send the identification information of the to-be-activated health monitoring equipment to the server, so that the server will verify the identification of the to-be-activated health monitoring equipment;
a first receiving unit 103, used to receive the authentication information of the to-be-activated health monitoring equipment sent from the server, wherein the authentication information of the to-be-activated health monitoring equipment is sent after the server verifies the identification of the to-be-activated health monitoring equipment;
an authentication unit 104, used to execute bidirectional authentication with the to-be-activated health monitoring equipment based on the authentication information of the to-be-activated health monitoring equipment and connect to the to-be-activated health monitoring equipment after successful authentication to activate the to-be-activated health monitoring equipment; and
a second transmitting unit 105, used to send the health monitoring equipment activation success information to the server, so that the server will mark the status of the health monitoring equipment as "activated"; and disconnect from the health monitoring equipment.

In one embodiment, the authentication information of the to-be-activated health monitoring equipment includes the key set corresponding to the to-be-activated health monitoring equipment, and the key set includes the keys and key identification corresponding to the to-be-activated health monitoring equipment;

The authentication unit 104 is specifically used to generate the third public key and the third private key according to the preset key negotiation algorithm; select any third key from the key set, and generate the fifth signature information by using the Hash algorithm according to the third key selected, the third time information and the third public key; send the third authentication request to the to-be-activated health monitoring equipment, wherein the third authentication request contains the identification information of the third key, the third time information, the third public key and the fifth signature information so that the to-be-activated health monitoring equipment will generate the sixth signature information by using the Hash algorithm according to the key corresponding to the identification of the third key, the third time information and the third public key and thus verify whether the sixth signature information matches the fifth signature information; receive the fourth authentication request that is sent when the to-be-activated health monitoring equipment confirms the sixth signature information matches the fifth signature information, wherein the fourth authentication request contains the identification information of the fourth key, the fourth time information, the fourth public key and the seventh signature information, wherein the seventh signature information is generated by the to-be-activated health monitoring equipment by using the Hash algorithm according to the fourth key selected, the fourth time information and the fourth public key, and the fourth public key is generated by the to-be-activated health monitoring equipment according to the preset key negotiation algorithm; generate the eighth signature information by using the Hash algorithm according to the key corresponding to the identification of the fourth key, the fourth time information and the fourth public key; and determine that the authentication is successful if the eighth signature information is confirmed to match the seventh signature information.

In one embodiment, the device further comprises:
a third transmitting unit, used to send the login request to the server before the identification information of the to-be-activated health monitoring equipment is sent to the server, wherein the login request contains the login information;
a second receiving unit, used to receive the access token information returned from the server, wherein the access token is generated by the server after the login information is successfully verified.

Based on the same inventive concept, the embodiment of the present application further provides a data transmission method implemented at the health monitoring equipment. As the principle of the data transmission method implemented at the health monitoring equipment to solve a problem is similar to that of the aforementioned data transmission method, the data transmission method implemented at the health monitoring equipment may be implemented by reference to the implementation of the aforementioned data transmission method, which is not described to avoid repetition.

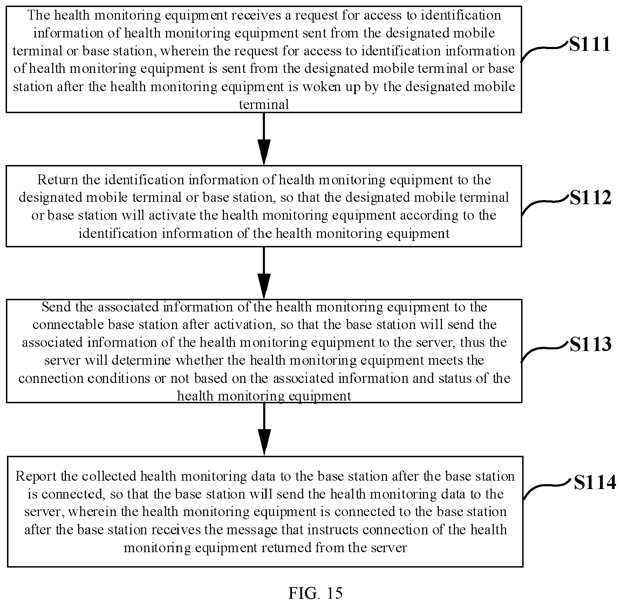

FIG. 15 illustrates a schematic diagram for the implementation process of the data transmission method implemented at the health monitoring equipment as provided in the embodiment of the present application, which may comprise the following steps:
S111. The health monitoring equipment receives a request for access to identification information of health monitoring equipment sent from the designated mobile terminal or base station, wherein the request for access to identification information of health monitoring equipment is sent from the designated mobile terminal or base station after the health monitoring equipment is woken up by the designated mobile terminal.

S 112. Return the identification information of health monitoring equipment to the designated mobile terminal or base station, so that the designated mobile terminal or base station will activate the health monitoring equipment according to the identification information of the health monitoring equipment.

S 113. Send the associated information of the health monitoring equipment to the connectable base station after activation, so that the base station will send the associated information of the health monitoring equipment to the server, thus the server will determine whether the health monitoring equipment meets the connection conditions or not based on the associated information and status of the health monitoring equipment.

S114. Report the collected health monitoring data to the base station after the base station is connected, so that the base station will send the health monitoring data to the server, wherein the health monitoring equipment is connected to the base station after the base station receives the message that instructs connection of the health monitoring equipment returned from the server.

In one embodiment, the message that instructs the connection of the health monitoring equipment contains the authentication information of the health monitoring equipment;
before connection with the base station, the method further comprises:
executing bidirectional authentication with the base station based on the authentication information of the health monitoring equipment;
confirming bidirectional authentication is successful.

In one embodiment, executing bidirectional authentication with the base station based on the authentication information of the health monitoring equipment, specifically comprising:
receiving the fifth authentication request sent from the base station, wherein the fifth authentication request contains the identification information of the fifth key, fifth time information, fifth public key and ninth signature information, the fifth public key is generated by the base station according to the preset key negotiation algorithm, the fifth key is selected by the base station from the key set contained in the authentication information of the health monitoring equipment returned from the server, and the ninth signature information is generated by the base station by using the Hash algorithm according to the fifth key, the fifth event information and the fifth public key;
generating the tenth signature information by using the Hash algorithm according to the key corresponding to the identification of the fifth key, the fifth time information and the fifth public key;
generating the sixth public key and sixth private key according to the preset key negotiation algorithm if the tenth signature information is confirmed to match the ninth signature information;
selecting any sixth key from the locally stored key set, and generating the eleventh signature information by using the Hash algorithm according to the selected sixth key, sixth time information and the sixth public key;
sending the sixth authentication request to the base station, wherein the sixth authentication request contains the identification information of the sixth key, the sixth time information, the sixth public key and the eleventh signature information, so that the base station will generate the twelfth signature information by using the Hash algorithm according to the key corresponding to the identification of the sixth key, the sixth time information and the sixth public key, so as to verify whether the twelfth signature information matches the eleventh signature information.

In one embodiment, if bidirectional authentication is successful, the method further comprises:
co-generating the shared key with the base station by using the preset key negotiation algorithm based on the fifth public key, the fifth private key corresponding to the fifth public key, the sixth public key and the sixth private key;
sending the shared key to the server, so that the server will store the correspondence between the identification information of the health monitoring equipment and the shared key; and
reporting the collected health monitoring data to the base station, so that the base station will send the health monitoring data to the server, specifically comprising:
   encrypting the health monitoring data according to the shared key and preset encryption algorithm, to generate the encrypted health monitoring data;
   sending the encrypted health monitoring data to the server, so that the server will decrypt the encrypted health monitoring data according to the shared key.

Based on the same inventive concept, the embodiment of the present application further provides a data transmission device implemented at the health monitoring equipment. As the principle of the data transmission device implemented at the health monitoring equipment to solve a problem is similar to that of the aforementioned data transmission method, the data transmission device implemented at the health monitoring equipment may be implemented by reference to the implementation of the aforementioned data transmission method, which is not described to avoid repetition.

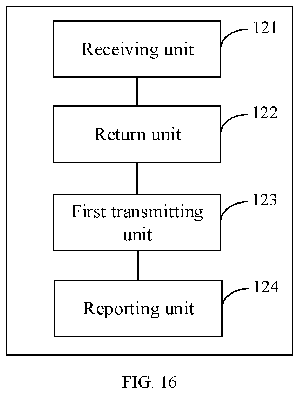

FIG. 16 illustrates a structural schematic diagram for the data transmission device implemented at the health monitoring equipment as provided in the embodiment of the present application, which may comprise:
a receiving unit 121, used to receive the request for access to identification information of health monitoring equipment sent from the designated mobile terminal or base station, wherein the request for access to identification information of health monitoring equipment is sent from the designated mobile terminal or the base station after the health monitoring equipment is woken up by the designated mobile terminal;
a return unit 122, used to return the identification information of the health monitoring equipment to the designated mobile terminal or the base station, so that the designated mobile terminal or the base station will activate the health monitoring equipment according to the identification information of the health monitoring equipment;
a first transmitting unit 123, used to send the associated information of the health monitoring equipment to the connectable base station after activation so that the base station will send the associated information of the health monitoring equipment to the server, thus the server will determine whether the health monitoring equipment meets the connection conditions or not based on the associated information and status of the health monitoring equipment; and
a reporting unit 124, used to report the collected health monitoring data to the base station after the base station is connected, so that the base station will send the health monitoring data to the server, wherein the health monitoring equipment is connected to the base station after the base station receives the message that instructs connection of the health monitoring equipment returned from the server.

In one embodiment, the message that instructs the connection of the health monitoring equipment contains the authentication information of the health monitoring equipment;
the device further comprising:
an authentication unit, used to execute bidirectional authentication with the base station based on the authentication information of the health monitoring equipment before the base station is connected;
a determination unit, used to determine bidirectional authentication is successful.

In one embodiment, the authentication unit is specifically used to receive the fifth authentication request sent from the base station, wherein the fifth authentication request contains the identification information of the fifth key, fifth time information, fifth public key and ninth signature information, the fifth public key is generated by the base station according to the preset key negotiation algorithm, the fifth key is selected by the base station from the key set contained in the authentication information of the health monitoring equipment returned from the server, and the ninth signature information is generated by the base station by using the Hash algorithm according to the fifth key, the fifth event information and the fifth public key; generate the tenth signature information by using the Hash algorithm according to the key corresponding to the fifth key, the fifth time information and the fifth public key; generate the sixth public key and sixth private key according to the preset key negotiation algorithm if the tenth signature information is confirmed to match the ninth signature information; selecting any sixth key from the locally stored key set, and generate the eleventh signature information by using the Hash algorithm according to the selected sixth key, sixth time information and sixth public key; send the sixth authentication request to the base station, wherein the sixth authentication request contains the identification information of the sixth key, the sixth time information, the sixth public key and the eleventh signature information, so that the base station will generate the twelfth signature information by using the Hash algorithm according to the key corresponding to the identification of the sixth key, the sixth time information and the sixth public key, so as to verify whether the twelfth signature information matches the eleventh signature information.

In one embodiment, further comprising:
a key negotiation unit, used to co-generate the shared key with the base station by using the preset key negotiation algorithm based on the fifth public key, the fifth private key corresponding to the fifth public key, the sixth public key and the sixth private key if bidirectional authentication is confirmed to be successful;
a second transmitting unit, used to send the shared key to the server, so that the server will store the correspondence between the identification information of the health monitoring equipment and the shared key; and
the reporting unit 124, specifically used to encrypt the health monitoring data according to the shared key and preset encryption algorithm, so as to generate encrypted health monitoring data; and send the encrypted health monitoring data to the server, so that the server will decrypt the encrypted health monitoring data according to the shared key.

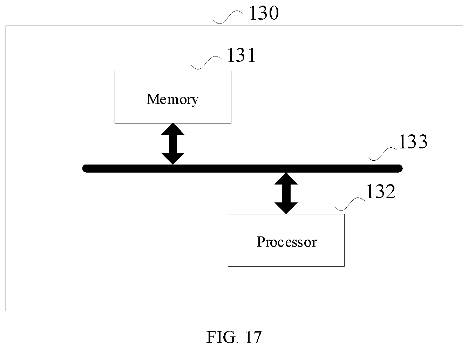

Based on the same technical concept, the embodiment of the present application further provides electronic equipment 130, as illustrated in FIG. 17. Electronic equipment 130 is used to implement the data transmission method recorded in the embodiment of the aforementioned method, and electronic equipment 130 of the embodiment may comprise: memory 131, processor 132 and a computer program that is saved in the memory and runs on the processor, e.g. a data transmission program. The processor implements the steps in the embodiments of the aforementioned data transmission methods while executing the computer program.

The embodiment of the present application does not define the specific connecting medium between the memory 131 and processor 132. The embodiment of the present application in FIG. 17 has memory 131 and processor 132 connected by bus 133, which is represented by a bold line in FIG. 17, and the connection method between other components is only illustrated schematically and not limited. The bus 133 may be divided into address bus, data bus, control bus, etc. For the convenience of representation, the bus is represented by only one bold line in FIG. 17, but this does not mean that there is only one bus or one type of bus.

Memory 131 may be a volatile memory, e.g. random access memory (RAM); memory 131 may also be a non-volatile memory, e.g. read-only memory, flash memory, hard disk drive (HDD) or solid state drive (SSD), or memory 131 is any other medium capable of carrying or storing expected program codes in the form of instructions or data structures and being accessed by a computer, but is not limited thereto. Memory 131 may be a combination of the memories mentioned above.

Processor 132 is used to implement the data transmission methods provided in the embodiments of the present application.

The embodiment of the present application further provides a computer-readable storage medium, which stores the computer-executable instructions that need to be executed to execute the aforesaid processor and comprises the program that needs to be executed to execute the aforesaid processor.

In some possible embodiments, all aspects of the data transmission methods provided in the present application may also be implemented in the form of a program product, which includes a program code. When the program product runs on electronic equipment, the program code is used to make the electronic equipment execute the steps in the data transmission methods according to various exemplary embodiments of the present application as described above in the present Description.

Those skilled in the art should understand that an embodiment of the present application may be provided as a method, a device or a computer program product. Accordingly, the present application may take the form of an entire hardware embodiment, an entire software embodiment or an embodiment combining software and hardware aspects. In addition, the present application may take the form of a computer program product implemented on one or more computer-usable storage media (including but not limited to disk memory, CD-ROM and optical memory) comprising a computer-usable program code.

The present application is described by reference to the flow diagrams and/or block diagrams according to the methods, equipment (devices) and computer program products in the embodiments of the present application. It should be understood that each flow and/or block in the flow diagrams and/or block diagrams and a combination thereof may be implemented by means of computer program instructions. These computer program instructions may be provided to a general-purpose computer, a special-purpose computer, an embedded processor or a processor of other programmable data processing equipment to produce a machine, such that the instructions, which are executed via the computer or the processor of other programmable data processing equipment, create means for implementing the functions specified in one or more flows of a flow diagram and/or one or more blocks of a block diagram.

These computer program instructions may also be stored in a computer-readable memory that may guide a computer or other programmable data processing equipment to work in a particular way, so that the instructions stored in the computer-readable memory generate a manufactured product including instruction means that implements the functions specified in one or more flows of a flow diagram and/or one or more blocks of a block diagram.

These computer program instructions may also be loaded on a computer or other programmable data processing equipment, to execute a series of operating steps on the computer or other programmable equipment to produce computer-implemented processing, so that instructions executed on the computer or other programmable equipment provide steps configured to implement functions specified in one or more flows of a flow diagram and/or one or more blocks of a block diagram.

Although the preferred embodiments of the present application have been described, those skilled in the art may make additional changes and modifications to these embodiments once they know the basic inventive concepts. Therefore, the appended claims are intended to be interpreted as covering the preferred embodiments and all changes and modifications falling within the scope of the present application.

Apparently, those skilled in the art may make various modifications and variations to the present application without departing from the spirit and scope of the present application. Therefore, the present application is intended to cover these modifications and variations provided that such modifications and variations made to the present application fall within the scope of the claims of the present application and equivalent technologies thereof.

## Claims

1. A data transmission method executed by server, **characterized by** comprising:
receiving health monitoring equipment activation success information and marking the status of the health monitoring equipment as "activated", wherein the health monitoring equipment activation success information is transmitted by the health monitoring equipment after the designated mobile terminal activates the health monitoring equipment, or transmitted after the base station activates the health monitoring equipment;
receiving the associated information of the candidate health monitoring equipment to be connected, wherein the candidate health monitoring equipment to be connected is connectable health monitoring equipment to be connected detected by the base station within its coverage;
returning to the base station a message that instructs the connection of the candidate health monitoring equipment to be connected if the connection conditions are determined to be met based on the associated information of the candidate health monitoring equipment to be connected and the status of the candidate health monitoring equipment to be connected, so that the base station will be connected to the candidate health monitoring equipment to be connected; and
receiving the health monitoring data that is transmitted from the base station and collected from the health monitoring equipment connected to the base station.

2. A method according to claim 1, **characterized in that**, the associated information of the candidate health monitoring equipment to be connected contains the identification information of the candidate health monitoring equipment to be connected and the information on signal strength between the candidate health monitoring equipment to be connected and the base station;
determining whether the connection conditions are met based on the associated information of the candidate health monitoring equipment to be connected and the status of the candidate health monitoring equipment to be connected, specifically comprising:
searching for the status of the candidate health monitoring equipment to be connected based on the identification of the candidate health monitoring equipment to be connected; and
confirming the connection conditions are met if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the first base station at the moment the equipment was disconnected from the base station to which it was connected last time.

3. A method according to claim 1, **characterized in that**, the associated information of the candidate health monitoring equipment to be connected contains the identification information of the candidate health monitoring equipment to be connected and the information on signal strength between the candidate health monitoring equipment to be connected and the base station;
determining whether the connection conditions are met based on the associated information of the candidate health monitoring equipment to be connected and the status of the candidate health monitoring equipment to be connected, specifically comprising:
searching for the status of the candidate health monitoring equipment to be connected based on the identification of the candidate health monitoring equipment to be connected; and
confirming the connection conditions are met if the status of the candidate health monitoring equipment to be connected is confirmed to be activated, and the signal strength between the candidate health monitoring equipment to be connected and the base station at the present moment is greater than the signal strength between the candidate health monitoring equipment to be connected and the second base station adjacent to the base station.

4. A method according to claim 1, **characterized in that**, the message that instructs the connection of the candidate health monitoring equipment contains the authentication information of the candidate health monitoring equipment to be connected, so that the base station and the candidate health monitoring equipment to be connected will execute bidirectional authentication based on the authentication information, and they will connect after successful authentication;
the authentication information of the candidate health monitoring equipment to be connected contains the key set corresponding to the candidate health monitoring equipment to be connected, and the key set contains the keys and key identification corresponding to the candidate health monitoring equipment to be connected.

5. A method according to claim 1, **characterized in that**, before the associated information of the candidate health monitoring equipment to be connected transmitted from the base station is received, further comprising:
receiving the login request sent from the base station, wherein the login request contains the identification information and login key information of the base station; and
verifying the identification information and login key information of the base station; returning the login token information to the base station if verification is passed, so that the base station will carry the login token when sending the data.

6. A data transmission method executed by base station, **characterized by** comprising:
sending the health monitoring equipment activation success information to the server after the to-be-activated health monitoring equipment is activated, so that the server will mark the status of the health monitoring equipment as "activated";
sending the associated information of the connectable candidate health monitoring equipment to be connected detected within the coverage to the server, so that the server will determine whether the connection conditions are met based on the associated information of the candidate health monitoring equipment to be connected and the status of the candidate health monitoring equipment to be connected;
connecting the candidate health monitoring equipment to be connected if the message that instructs the connection of the candidate health monitoring equipment to be connected is received from the server; and
receiving the health monitoring data reported from the connected health monitoring equipment, and sending the health monitoring data reported from the health monitoring equipment to the server.

7. A method according to claim 6, **characterized in that**, the to-be-activated health monitoring equipment will be activated by:
receiving the Bluetooth broadcast data packet sent from the to-be-activated health monitoring equipment, wherein the Bluetooth broadcast data packet is sent after the to-be-activated health monitoring equipment is woken up by the designated mobile terminal;
obtaining the identification information of the to-be-activated health monitoring equipment from the to-be-activated health monitoring equipment;
sending the identification information of the to-be-activated health monitoring equipment to the server, so that the server will verify the identification information of the to-be-activated health monitoring equipment;
receiving the authentication information of the to-be-activated health monitoring equipment sent from the server, wherein the authentication information of the to-be-activated health monitoring equipment is sent after the server verifies the identification information of the to-be-activated health monitoring equipment; and
executing bidirectional authentication with the to-be-activated health monitoring equipment based on the authentication information of the to-be-activated health monitoring equipment, and activating the to-be-activated health monitoring equipment after authentication is successful.

8. A method according to claim 7, **characterized in that**, the authentication information of the to-be-activated health monitoring equipment contains the key set corresponding to the to-be-activated health monitoring equipment, and the key set contains the keys and key identification corresponding to the to-be-activated health monitoring equipment;
executing bidirectional authentication with the to-be-activated health monitoring equipment based on the authentication information of the to-be-activated health monitoring equipment, specifically comprising:
generating the first public key and first private key according to the preset key negotiation algorithm;
selecting any first key from the key set, and generating the first signature information by using the Hash algorithm according to the selected first key, first time information and the first public key;
sending the first authentication request to the to-be-activated health monitoring equipment, wherein the first authentication request contains the identification information of the first key, the first time information, the first public key and the first signature information, so that the to-be-activated health monitoring equipment will generate the second signature information by using the Hash algorithm according to the key corresponding to the identification of the first key, the first time information and the first public key, so as to verify whether the second signature information matches the first signature information;
receiving the second authentication request sent when the to-be-activated health monitoring equipment confirms the second signature information matches the first signature information, wherein the second authentication request contains the identification information of the second key, second time information, second public key and third signature information, the third signature information is generated by the to-be-activated health monitoring equipment by using the Hash algorithm according to the selected second key, the second time information and the second public key and the second public key is generated by the to-be-activated health monitoring equipment by using the preset key negotiation algorithm;
generating the fourth signature information by using the Hash algorithm according to the key corresponding to the identification of the second key, the second time information and the second public key; and
confirming the authentication is successful if the fourth signature information is confirmed to match the third signature information.

9. A data transmission method executed by assigned mobile terminal, **characterized by** comprising:
waking up the to-be-activated health monitoring equipment, and obtaining the identification information of the to-be-activated health monitoring equipment;
sending the identification information of the to-be-activated health monitoring equipment to the server, so that the server will verify the identification of the to-be-activated health monitoring equipment;
receiving the authentication information of the to-be-activated health monitoring equipment sent from the server, wherein the authentication information of the to-be-activated health monitoring equipment is sent after the server verifies the identification of the to-be-activated health monitoring equipment;
executing bidirectional authentication with the to-be-activated health monitoring equipment based on the authentication information of the to-be-activated health monitoring equipment, wherein, after authentication is successful, the to-be-activated health monitoring equipment will be connected and the to-be-activated health monitoring equipment will be activated; and
sending the health monitoring equipment activation success information to the server, so that the server will mark the status of the health monitoring equipment as "activated"; disconnecting from the health monitoring equipment.

10. A method according to claim 9, **characterized in that**, the authentication information of the to-be-activated health monitoring equipment contains the key set corresponding to the to-be-activated health monitoring equipment, and the key set contains the keys and key identification corresponding to the to-be-activated health monitoring equipment;
executing bidirectional authentication with the to-be-activated health monitoring equipment based on the authentication information of the to-be-activated health monitoring equipment, specifically comprising:
generating the third public key and third private key according to the preset key negotiation algorithm;
selecting any third key from the key set, and generating the fifth signature information by using the Hash algorithm according to the selected third key, third time information and the third public key;
sending the third authentication request to the to-be-activated health monitoring equipment, wherein the third authentication request contains the identification information of the third key, the third time information, the third public key and the fifth signature information, so that the to-be-activated health monitoring equipment will generate the sixth signature information by using the Hash algorithm according to the key corresponding to the identification of the third key, the third time information and the third public key, so as to verify whether the sixth signature information matches the fifth signature information;
receiving the fourth authentication request sent when the to-be-activated health monitoring equipment confirms the sixth signature information matches the fifth signature information, wherein the fourth authentication request contains the identification information of the fourth key, fourth time information, fourth public key and seventh signature information, the seventh signature information is generated by the to-be-activated health monitoring equipment by using the Hash algorithm according to the selected fourth key, the fourth time information and the fourth public key, and the fourth public key is generated by the to-be-activated health monitoring equipment according to the preset key negotiation algorithm;
generating the eighth signature information by using the Hash algorithm according to the key corresponding to the identification of the fourth key, the fourth time information and the fourth public key; and
confirming the authentication is successful if the eighth signature information is confirmed to match the seventh signature information.

11. A method according to claim 9, **characterized by**, before the identification information of the to-be-activated health monitoring equipment is sent to the server, further comprising:
sending a login request to the server, wherein the login request contains the login information; and
receiving the access token information returned from the server, wherein the access token is generated by the server after the login information is successfully verified.

12. A data transmission method executed by health monitoring equipment, **characterized by** comprising:
receiving the request for access to identification information of health monitoring equipment sent from the designated mobile terminal or base station, wherein the request for access to identification information of health monitoring equipment is sent from the designated mobile terminal or base station after the health monitoring equipment is woken up by the designated mobile terminal;
returning the identification information of the health monitoring equipment to the designated mobile terminal or the base station, so that the designated mobile terminal or the base station will activate the health monitoring equipment according to the identification information of the health monitoring equipment;
sending the associated information of the health monitoring equipment to the connectable base station after activation, so that the base station will send the associated information of the health monitoring equipment to the server, and the server will determine whether the connection conditions are met based on the associated information of the health monitoring equipment and the status of the health monitoring equipment; and
reporting the collected health monitoring data to the base station after connecting the base station, so that the base station will send the health monitoring data to the server, wherein the health monitoring equipment is connected to the base station after the base station receives the message that instructs the connection of the health monitoring equipment returned from the server.

13. A method according to claim 12, **characterized in that**, the message that instructs the connection of the health monitoring equipment contains the authentication information of the health monitoring equipment;
before connection with the base station, the method further comprises:
executing bidirectional authentication with the base station based on the authentication information of the health monitoring equipment; and
confirming bidirectional authentication is successful;
bidirectional authentication will be executed with the base station based on the authentication information of the health monitoring equipment, specifically comprising:
receiving the fifth authentication request sent from the base station, wherein the fifth authentication request contains the identification information of the fifth key, fifth time information, fifth public key and ninth signature information, the fifth public key is generated by the base station according to the preset key negotiation algorithm, the fifth key is selected by the base station from the key set contained in the authentication information of the health monitoring equipment returned from the server, and the ninth signature information is generated by the base station by using the Hash algorithm according to the fifth key, the fifth event information and the fifth public key;
generating the tenth signature information by using the Hash algorithm according to the key corresponding to the identification of the fifth key, the fifth time information and the fifth public key;
generating the sixth public key and sixth private key according to the preset key negotiation algorithm if the tenth signature information is confirmed to match the ninth signature information;
selecting any sixth key from the locally stored key set, and generating the eleventh signature information by using the Hash algorithm according to the selected sixth key, sixth time information and the sixth public key; and
sending the sixth authentication request to the base station, wherein the sixth authentication request contains the identification information of the sixth key, the sixth time information, the sixth public key and the eleventh signature information, so that the base station will generate the twelfth signature information by using the Hash algorithm according to the key corresponding to the identification of the sixth key, the sixth time information and the sixth public key, so as to verify whether the twelfth signature information matches the eleventh signature information.

14. A method according to claim 13, **characterized by**, if bidirectional authentication is confirmed to be successful, further comprising:
co-generating the shared key with the base station by using the preset key negotiation algorithm based on the fifth public key, the fifth private key corresponding to the fifth public key, the sixth public key and the sixth private key;
sending the shared key to the server, so that the server will store the correspondence between the identification information of the health monitoring equipment and the shared key; and
reporting the collected health monitoring data to the base station, so that the base station will send the health monitoring data to the server, specifically comprising:
encrypting the health monitoring data according to the shared key and preset encryption algorithm, to generate the encrypted health monitoring data; and
sending the encrypted health monitoring data to the server, so that the server will decrypt the encrypted health monitoring data according to the shared key.

15. An electronic equipment comprising memory, processor and the computer program that is stored on the memory and operable on the processor. The electronic equipment, **characterized in that** the data transmission method described in any one of claims 1~14 is implemented when the processor executes the program.

16. A computer-readable storage medium, in which the computer program is stored, **characterized in that** the steps of the data transmission method described in any one of claims 1~14 are implemented when the program is executed by the processor.
